# EUROPEAN PATENT SPECIFICATION

(11) **EP 3 094 346 B1**
(45) Date of publication and mention of the grant of the patent: **11.12.2019**
(21) Application number: 15737353.1
(22) Date of filing: 23.01.2015
(51) Int. Cl.: A61K 39/00, A61K 39/395, A61K 38/00, A61K 38/10, G01N 33/566, C07K 16/28, C07K 7/08

(54) **VISTA ANTAGONIST AND METHODS OF USE**
VISTA-ANTAGONISTEN UND VERFAHREN ZUR VERWENDUNG
ANTAGONISTE DE VISTA ET PROCÉDÉS D'UTILISATION

(30) Priority: 14.01.2014 US 201461927061 P; 06.11.2014 US 201414534793
(43) Date of publication of application: 23.11.2016
(73) Proprietor: The Trustees Of Dartmouth College, Hanover, NH 03755 (US); King's College London, London WC2R 2LS (GB); Spaller, Mark, Lebanon, New Hampshire 03756 (US)
(72) Inventor: NOELLE, Randolph, J., Plainfield, New Hampshire 03781 (US); CEERAZ, Sabrina, Lebanon, New Hampshire 03766 (US); LEMERCIER, Isabelle, Enfield, New Hampshire 03748 (US); NOWAK, Elizabeth, West Lebanon, New Hampshire 03784 (US); LINES, Janet, London SE1 4JZ (GB); WANG, Li, Norwich, Vermont 05055 (US); SPALLER, Mark, Lebanon, New Hampshire 03756 (US)
(74) Representative: Stephens, Clare Jean
(86) International application number: PCT/US2015/012752
(87) International publication number: WO 2015/109340

(56) References cited:
- WO-A2-2011/120013
- US-A1- 2005 063 948
- LI WANG ET AL: "VISTA, a novel mouse Ig superfamily ligand that negatively regulates T cell responses", THE JOURNAL OF EXPERIMENTAL MEDICINE, vol. 208, no. 3, 7 March 2011 (2011-03-07) , pages 577-592, XP055387118, US ISSN: 0022-1007, DOI: 10.1084/jem.20100619
- XU ET AL.: 'The genomic sequence of the Chinese hamster ovary (CHO)-K1 cell line.' NATURE BIOTECHNOLOGY vol. 29, no. 8, August 2011, ISSN 1087-0156 pages 735 - 741, XP055223996
- WANG ET AL.: 'VISTA, a novel mouse Ig superfamily ligand that negatively regulates T cell responses.' J. EXP. MED. vol. 208, no. 3, 07 March 2011, ISSN 0022-1007 pages 577 - 592, XP002738331
- WANG ET AL.: 'Immune checkpoint protein VISTA regulate autoimmunity and anti-tumor immunity' J IMMUNOL vol. 190, no. 1 SUPP, 53.35, 01 May 2013, XP055357336
- LI WANG ET AL: "VISTA, a novel mouse Ig superfamily ligand that negatively regulates T cell responses", THE JOURNAL OF EXPERIMENTAL MEDICINE, vol. 208, no. 3, 7 March 2011 (2011-03-07) , pages 577-592, XP055387118, US ISSN: 0022-1007, DOI: 10.1084/jem.20100619

### SEQUENCE LISTING

<110> Noelle, Randolph
   Ceeraz, Sabrina
   LeMercier, Isabelle
   Nowak, Elizabeth
   Lines, Janet
   Wang, Li
   Spaller, Mark
<120> VISTA Antagonist and Methods of Use
<130> 43260.1003
<150> 13/925,094
   <151> 2013-06-24
<150> 61/663,969
   <151> 2012-06-25
<150> 61/663,431
   <151> 2012-06-22
<150> 61/927,061
   <151> 2014-01-14
<160> 1
<170> PatentIn version 3.5
<210> 1
   <211> 12
   <212> PRT
   <213> Artificial Sequence
<220>
   <223> AP1049
<400> 1
   Ser Ser Ala Cys Asp Trp Ile Lys Arg Ser Cys His
   1               5                   10

## Description

### Background

The immune system is tightly controlled by co-stimulatory and co-inhibitory ligands and receptors. These molecules provide not only a second signal for T cell activation but also a balanced network of positive and negative signals to maximize immune responses against infection while limiting immunity to self.

Induction of an immune response requires T cell expansion, differentiation, contraction and establishment of T cell memory. T cells must encounter antigen presenting cells (APCs) and communicate via T cell receptor (TCR)/major histocompatibility complex (MHC) interactions on APCs. Once the TCR/MHC interaction is established, other sets of receptor-ligand contacts between the T cell and the APC are required, i.e. co-stimulation via CD154/CD40 and CD28/B7.1-B7.2. The synergy between these contacts results in a productive immune response capable of clearing pathogens and tumors, and may be capable of inducing autoimmunity.

Another level of control has been identified, namely regulatory T cells (T_{reg}). This specific subset of T cells is generated in the thymus, delivered into the periphery, and is capable of constant and inducible control of T cells responses. Sakaguchi (2000) Cell 101(5):455-8; Shevach (2000) Annu. Rev. Immunol. 18:423-49; Bluestone and Abbas (2003) Nat. Rev. Immunol. 3(3):253-7. T_{reg} are represented by a CD4+CD25+ phenotype and also express high levels of cytotoxic T lymphocyte-associated antigen-4 (CTLA-4), OX-40, 4-1BB and the glucocorticoid inducible TNF receptor-associated protein (GITR). McHugh, et al. (2002) Immunity 16(2):311-23; Shimizu, et al. (2002) Nat. Immun. 3(2):135-42. Elimination of T_{reg} cells by 5 day neonatal thymectomy or antibody depletion using anti-CD25, results in the induction of autoimmune pathology and exacerbation of T cells responses to foreign and self-antigens, including heightened anti-tumor responses. Sakaguchi, et al. (1985) J. Exp. Med. 161(1):72-87; Sakaguchi, et al. (1995) J. Immunol. 155(3):1151-64; Jones, et al. (2002) Cancer Immun. 2:1. In addition, T_{reg} have also been involved in the induction and maintenance of transplantation tolerance, since depletion of T_{reg} with anti-CD25 monoclonal antibodies results in ablation of transplantation tolerance and rapid graft rejection. Jarvinen, et al. (2003) Transplantation 76:1375-9. Among the receptors expressed by T_{reg} GITR seems to be an important component since ligation of GITR on the surface of Treg with an agonistic monoclonal antibody results in rapid termination of T_{reg} activity, resulting in autoimmune pathology and ablation of transplantation tolerance.

Costimulatory and co-inhibitory ligands and receptors not only provide a "second signal" for T cell activation, but also a balanced network of positive and negative signal to maximize immune responses against infection while limiting immunity to self. The best characterized costimulatory ligands are B7.1 and B7.2, which are expressed by professional APCs, and whose receptors are CD28 and CTLA-4. Greenwald, et al. (2005) Annu Rev Immunol 23, 515-548; Sharpe and Freeman (2002) Nat Rev Immunol 2, 116-126. CD28 is expressed by naive and activated T cells and is critical for optimal T cell activation. In contrast, CTLA-4 is induced upon T cell activation and inhibits T cell activation by binding to B7.1/B7.2, thus impairing CD28-mediated costimulation. CTLA-4 also transduces negative signaling through its cytoplasmic ITIM motif. Teft, et al. (2006). Annu Rev Immunol 24, 65-97. B7.1/B7.2 KO mice are impaired in adaptive immune response (Borriello, et al. (1997) Immunity 6, 303-313; Freeman, et al. (1993) Science 262, 907-909), whereas CTLA-4 KO mice can not adequately control inflammation and develop systemic autoimmune diseases. Chambers, et al. (1997) Immunity 7, 885-895; Tivol, et al. (1995) Immunity 3, 541-547; Waterhouse, et al. (1995) Science 270, 985-988. The B7 family ligands have expanded to include costimulatory B7-H2 (ICOS Ligand) and B7-H3, as well as co-inhibitory B7-H1 (PD-L1), B7-DC (PD-L2), B7-H4 (B7S1 or B7x), and B7-H6. *See* Brandt, et al. (2009) J Exp Med 206, 1495-1503; Greenwald, et al. (2005) Annu Rev Immunol 23: 515-548.

Inducible costimulatory (ICOS) molecule is expressed on activated T cells and binds to B7-H2. *See* Yoshinaga, et al. (1999) Nature 402, 827-832. ICOS is important for T cell activation, differentiation and function, as well as essential for T-helper-cell-induced B cell activation, Ig class switching, and germinal center (GC) formation. Dong, et al. (2001) Nature 409, 97-101; Tafuri, et al. (2001) Nature 409, 105-109; Yoshinaga, et al. (1999) Nature 402, 827-832. Programmed Death 1 (PD-1) on the other hand, negatively regulates T cell responses. PD-1 KO mice develop lupus-like autoimmune disease, or autoimmune dilated cardiomyopathy depending upon the genetic background. Nishimura, et al. (1999) Immunity 11, 141-151. Nishimura, et al. (2001) Science 291: 319-322. The autoimmunity most likely results from the loss of signaling by both ligands PP-L1 and PD-L2. Recently, CD80 was identified as a second receptor for PP-L1 that transduces inhibitory signals into T cells. Butte, et al. (2007) Immunity 27: 111-122. The receptor for B7-H3 and B7-H4 still remain unknown.

The best characterized co-stimulatory ligands are B7.1 and B7.2, which belong to the Ig superfamily and are expressed on professional APCs and whose receptors are CD28 and CTLA-4 (Greenwald, et al. (2005) Annu. Rev. Immunol. 23:515-548). CD28 is expressed by naive and activated T cells and is critical for optimal T cell activation. In contrast, CTLA-4 is induced upon T cell activation and inhibits T cell activation by binding to B7.1/B7.2, impairing CD28-mediated co-stimulation. B7.1 and B7.2 KO mice are impaired in adaptive immune response (Borriello, et al. (1997) Immunity 6:303-313), whereas CTLA-4 knockout mice cannot adequately control inflammation and develop systemic autoimmune diseases (Tivol, et al. (1995) Immunity 3:541-547; Waterhouse, et al. (1995) Science 270:985-988; Chambers, et al. (1997) Immunity 7:885-895) .

The B7 family ligands have expanded to include co-stimulatory B7-H2 (inducible T cell co-stimulator (ICOS) ligand) and B7-H3, as well as co-inhibitory B7-H1 (PD-L1), B7-DC (PD-L2), B7-H4 (B7S1 or B7x), and B7-H6 (Greenwald, et al. (2005) *supra;* Brandt, et al. (2009) J. Exp. Med. 206:1495-1503). Accordingly, additional CD28 family receptors have been identified. ICOS is expressed on activated T cells and binds to B7-H2 (Yoshinaga, et al. (1999) Nature 402:827-832). ICOS is a positive coregulator, which is important for T cell activation, differentiation, and function (Yoshinaga, et al. (1999) *supra;* Dong, et al. (2001) Nature 409:97-101). In contrast, PD-1 (programmed death 1) negatively regulates T cell responses. PD-1 knockout mice develop lupus-like autoimmune disease or autoimmune dilated cardiomyopathy (Nishimura, et al. (1999) Immunity 11:141-151; Nishimura, et al. (2001) Science 291:319-322). The autoimmunity most likely results from the loss of signaling by both ligands PD-L1 and PD-L2. Recently, CD80 was identified as a second receptor for PD-L1 that transduces inhibitory signals into T cells (Butte, et al. (2007) Immunity 27:111-122).

The two inhibitory B7 family ligands, PD-L1 and PD-L2, have distinct expression patterns. PD-L2 is inducibly expressed on DCs and macrophages, whereas PD-L1 is broadly expressed on both hematopoietic cells and nonhematopoietic cell types (Okazaki & Honjo (2006) Immunology 27:195-201; Keir, et al. (2008) Annu. Rev. Immunol. 26:677-704). Consistent with the immune-suppressive role of PD-1 receptor, a study using PD-L1^{-/-} and PD-L2^{-/-} mice has shown that both ligands have overlapping roles in inhibiting T cell proliferation and cytokine production (Keir, et al. (2006) J. Exp. Med. 203:883-895). PP-L1 deficiency enhances disease progression in both the non-obese diabetic model of autoimmune diabetes and the mouse model of multiple sclerosis (experimental autoimmune encephalomyelitis (EAE); Anasari, et al. (2003) J. Exp. Med. 198:63-69; Salama, et al. (2003) J. Exp. Med. 198:71-78; Latchman, et al. (2004) Proc. Natl. Acad. Sci. USA. 101:10691-10696). PD-L1^{-/-} T cells produce elevated levels of the proinflammatory cytokines in both disease models. In addition, bone marrow chimera experiments have demonstrated that the tissue expression of PP-L1 (*i.e*., within pancreas) uniquely contributes to its capacity of regionally controlling inflammation (Keir, et al. (2006) *supra;* Keir, et al. (2007) J. Immunol. 179:5064-5070; Grabie, et al. (2007) Circulation. 116:2062-2071). PP-L1 is also highly expressed on placental syncytiotrophoblasts, which critically control the maternal immune responses to allogeneic fetus (Guleria, et al. (2005) J. Exp. Med. 202:231-237).

Consistent with its immune-suppressive role, PP-L1 potently suppresses antitumor immune responses and helps tumors evade immune surveillance. PP-L1 can induce apoptosis of infiltrating cytotoxic CD8⁺ T cells, which express a high level of PD-1 (Dong, et al. (2002) Nature 409:97-101; Dong & Chen (2003) J. Mol. Med. 81:281-287). Studies have shown that blocking the PD-L1-PD-1 signaling pathway, in conjunction with other immune therapies, prevents tumor progression by enhancing antitumor cytotoxic T lymphocyte activity and cytokine production (Iwai, et al. (2002) Proc. Natl. Acad. Sci. USA 99:12293-12297; Blank, et al. (2004) Cancer Res. 64:1140-1145; Blank, et al. (2005) Cancer Immunol. Immunother. 54:307-314; Geng, et al. (2006) Int. J. Cancer. 118:2657-2664). In addition, it has been shown that PD-L1 expression on dendritic cells promotes the induction of adaptive Foxp3⁺CD4⁺ regulatory T cells (aT_{reg} cells), and PD-L1 is a potent inducer of aT_{reg} cells within the tumor microenvironment (Wang, et al. (2008) Proc. Natl. Acad. Sci. USA. 105:9331-9336).

An additional immune regulatory ligand, referred to as V-domain Ig suppressor of T cell activation (VISTA) or PD-L3, has been recently identified as an upregulated molecule in a T cell transcriptional profiling screen. (Wang, et al. (2011) J. Exp. Med. 208:577; WO 2011/120013). It has been shown that the extracellular Ig domain of VISTA shares significant sequence homology with the B7 family ligands PD-L1 and PD-L2, albeit with unique structural features that distinguish it from the B7 family members.

VISTA is primarily expressed on hematopoietic cells, and VISTA expression is highly regulated on myeloid antigen-presenting cells (APCs) and T cells. Expression of VISTA on antigen presenting cells (APCs) suppresses T cell responses by engaging its counter-receptor on T cells during cognate interactions between T cells and APCs. VISTA blockade enhances T cell-mediated immunity in an autoimmune disease model, suggesting its unique and non-redundant role in controlling autoimmunity when compared with other inhibitory B7 family ligands such as PD-L1 and PD-L2. In addition, VISTA blockade enhances anti-tumor immunity and suppressed tumor growth in preclinical murine tumor models (WO 2011/120013). In this regard, therapeutic intervention of the VISTA inhibitory pathway represents a novel approach to modulate T cell-mediated immunity for treating diseases such as viral infection and cancer.

### Summary of the Invention

The present invention provides an isolated VISTA antagonist that comprises a peptide that is identical to the amino acid sequence of SEQ ID NO:1 (Ser-Ser-Ala-Cys-Asp-Trp-Ile-Lys-Arg-Ser-Cys-His), or a multimer, conjugate,
or derivative thereof, wherein the derivative thereof contains a modification of one or more amino acid residues or a linker group or other covalently linked group.

In one embodiment, the isolated VISTA antagonist comprises a peptide which is identical to the amino acid sequence of SEQ ID NO:1 (Ser-Ser-Ala-Cys-Asp-Trp-Ile-Lys-Arg-Ser-Cys-His), or which comprises a peptide having an amino acid sequence that differs from SEQ ID NO:1 by at most 2 amino acid residues, or a multimer, conjugate, or derivative thereof, wherein the derivative thereof contains a modification of one or more amino acid residues or a linker group or other covalently linked group. In another embodiment, the isolated VISTA antagonist comprises a peptide having an amino acid sequence that differs from SEQ ID NO:1 by at most 1 amino acid residue, or a multimer, conjugate, or derivative thereof, wherein the derivative thereof contains a modification of one or more amino acid residues or a linker group or other covalently linked group. In yet another embodiment, the isolated VISTA antagonist comprises a peptide which is identical to the amino acid sequence of SEQ ID NO:1 (Ser-Ser-Ala-Cys-Asp-Trp-Ile-Lys-Arg-Ser-Cys-His), or a multimer, or conjugate thereof. In a specific embodiment, the isolated VISTA antagonist consists of the amino acid sequence of SEQ ID NO:1 (Ser-Ser-Ala-Cys-Asp-Trp-Ile-Lys-Arg-Ser-Cys-His).

In one embodiment, the cysteine residues at positions 4 and 11 of SEQ ID NO:1 (Ser-Ser-Ala-Cys-Asp-Trp-Ile-Lys-Arg-Ser-Cys-His), or their corresponding positions in a variant of said peptide, form a disulfide bridge.

In another embodiment, the isolated VISTA antagonist has been modified to improve binding affinity and/or stability. In a specific embodiment, the isolated VISTA antagonist has been modified by PEG, acetylation, XTEN, albumin and/or multimerization.

In another embodiment, the isolated VISTA antagonist is directly or indirectly attached to an immunoglobulin polypeptide or a fragment thereof. The immunoglobulin polypeptide may comprise a human IgG1, IgG2, IgG3 or IgG4 constant region or fragment thereof. Preferably, the immunoglobulin polypeptide comprises a human IgG1 constant region or fragment thereof.

In yet another embodiment, the isolated VISTA antagonist comprises multiple, i.e., 2, 3, 4, 5, 6, 7 or more, copies of said peptide.

In a further embodiment, the isolated VISTA antagonist comprises another moiety that targets said peptide to a target site. The targeting moiety may be selected from an antibody or ligand that binds to an antigen, a receptor expressed by the target cell or an infectious agent.

In yet a further embodiment, the isolated VISTA antagonist is attached to another moiety or another copy of said antagonist via a linker. The linker may be a peptide that permits the antagonist to interact with VISTA expressed on the surface of a target cell.

In a further embodiment, the isolated VISTA antagonist is directly or indirectly attached to a detectable label or therapeutic agent.

In several of the embodiments, the isolated VISTA antagonist binds to the extracellular domain of VISTA and disrupts its interaction with a VISTA receptor and/or reduces or inhibits VISTA-mediated T cell suppression.

In one embodiment, the isolated VISTA antagonist elicits anti-tumor and/or anti-viral activity.

Additionally, the invention contemplates a composition suitable for therapeutic, prophylactic or diagnostic use comprising a therapeutically, prophylactically or diagnostically effective amount of the isolated VISTA antagonist.

In one embodiment, the composition further comprises a pharmaceutically acceptable carrier, diluent, solubilizer, preservative or mixture thereof.

In another embodiment, the composition further comprises another therapeutic agent, e.g., an anti-cancer agent, an anti-viral agent, a cytokine or an immune agonist. In a particular embodiment, the other therapeutic agent is selected from CTLA-4-Ig, anti-PD-1, PD-L1 or PD-L2 fusion proteins, and EGFR antagonists.

In one embodiment, the composition is suitable for subcutaneous administration or intravenous administration.

Moreover, the present invention further contemplates an isolated nucleic acid sequence encoding a VISTA antagonist according to claim 1,
a vector containing the isolated nucleic acid sequence, and a host cell comprising the isolated nucleic acid sequence or the vector.

In one embodiment, the host cell is a mammalian cell, a bacterial cell, a fungal cell, a yeast cell, an avian cell or an insect cell.

The present invention further contemplates a method of expressing a VISTA antagonist
comprising culturing the host cell under conditions that provide for expression of said VISTA antagonist.

Furthermore, the present invention contemplates various uses of the isolated VISTA antagonist.

In one embodiment, the invention provides a VISTA antagonist according to claim 1, or a composition comprising an effective amount of a VISTA antagonist according to claim 1, for use in blocking, inhibiting or neutralizing VISTA-mediated T cell suppression.

In another embodiment, the invention provides a VISTA antagonist according to claim 1, or a composition comprising an effective amount of a VISTA antagonist according to claim 1, for use in stimulating an immune response in a subject in need thereof. The VISTA antagonist or composition may be for use in treating cancer in a subject.

The subject may have cancer and/or an infection selected from the group consisting of bacterial, viral, parasitic and fungal infections.

The bacterial infection may be caused by at least one bacterium selected from the group consisting of Bordetella, Borrelia, Brucella, Burkholderia, Campylobacter, Chlamydia, Clostridium, Corynebacterium, Enterobacter, Enterococcus, Erwinia, Escherichia, Francisella, Haemophilus, Heliobacter, Legionella, Leptospira, Listeria, Mycobacterium, Mycoplasma, Neisseria, Pasteurella, Pelobacter, Pseudomonas, Rickettsia, Salmonella, Serratia, Shigella, Staphylococcus, Streptococcus, Treponema, Vibrio, Yersinia and Xanthomonas.

The viral infection may be caused by at least one virus selected from the group consisting of Adenoviridae, Papillomaviridae, Polyomaviridae, Herpesviridae, Poxviridae, Hepadnaviridae, Parvoviridae, Astroviridae, Caliciviridae, Picornaviridae, Coronoviridae, Flaviviridae, Retroviridae, Togaviridae, Arenaviridae, Bunyaviridae, Filoviridae, Orthomyxoviridae, Paramyxoviridae, Rhabdoviridae, and Reoviridae. More specifically, the virus may be adenovirus, herpes simplex type I, herpes simplex type 2, Varicella-zoster virus, Epstein-barr virus, cytomegalovirus, herpesvirus type 8, papillomavirus, BK virus, JC virus, smallpox, Hepatitis B, bocavirus, parvovirus B19, astrovirus, Norwalk virus, coxsackievirus, Hepatitis A, poliovirus, rhinovirus, severe acute respiratory syndrome virus, Hepatitis C, yellow fever, dengue virus, West Nile virus, rubella, Hepatitis E, human immunodeficiency virus (HIV), influenza, guanarito virus, Junin virus, Lassa virus, Machupo virus, Sabia virus, Crimean-Congo hemorrhagic fever virus, ebola virus, Marburg virus, measles virus, mumps virus, parainfluenza, respiratory syncytial virus, human metapneumovirus, Hendra virus, Nipah virus, rabies, Hepatitis D, rotavirus, orbivirus, coltivirus or Banna virus.

The fungal infection may be selected from the group consisting of thrush, candidiasis, cryptococcosis, histoplasmosis, blastomycosis, aspergillosis, coccidioidomycosis, paracoccidiomycosis, sporotrichosis, zygomycosis, chromoblastomycosis, lobomycosis, mycetoma, onychomycosis, piedra pityriasis versicolor, tinea barbae, tinea capitis, tinea corporis, tinea cruris, tinea favosa, tinea nigra, tinea pedis, otomycosis, phaeohyphomycosis, or rhinosporidiosis.

The parasitic infection may be caused by at least one parasite selected from the group consisting of Entamoeba hystolytica, Giardia lamblia, Cryptosporidium muris, Trypanosomatida gambiense, Trypanosomatida rhodesiense, Trypanosomatida crusi, Leishmania mexicana, Leishmania braziliensis, Leishmania tropica, Leishmania donovani, Toxoplasma gondii, Plasmodium vivax, Plasmodium ovale, Plasmodium malariae, Plasmodium falciparum, Trichomonas vaginalis, Histomonas meleagridis; Secementea; Trichuris trichiura, Ascaris lumbricoides, Enterobius vermicularis, Ancylostoma duodenale, Necator americanus, Strongyloides stercoralis, Wuchereria bancrofti, Dracunculus medinensis; blood flukes, liver flukes, intestinal flukes, lung flukes; Schistosoma mansoni, Schistosoma haematobium, Schistosoma japonicum, Fasciola hepatica, Fasciola gigantica, Heterophyes heterophyes, and Paragonimus westermani.

In another embodiment, the invention provides a VISTA antagonist according to claim 1, or a composition comprising an effective amount of a VISTA antagonist according to claim 1, for use in enhancing anti-cancer or anti-tumor immunity, in a subject in need thereof.

In another embodiment, the invention provides a VISTA antagonist according to claim 1, or a composition comprising an effective amount of a VISTA antagonist according to claim 1, for use in treating or preventing cancer, inhibiting tumor invasion and/or cancer metastasis, in a subject in need thereof.

The cancer may be selected from the group consisting of carcinoma, lymphoma, blastoma, sarcoma, leukemia, lymphoid malignancies, melanoma, squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma, head and neck cancer, B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; post-transplant lymphoproliferative disorder (PTLD), abnormal vascular proliferation associated with phakomatoses, edema (such as that associated with brain tumors), and Meigs' syndrome.

In yet another embodiment, the invention provides a VISTA antagonist according to claim 1, or a composition comprising an effective amount of a VISTA antagonist according to claim 1, for use in treating or preventing a viral infection, in a subject in need thereof.

These uses may further comprise the use of another therapeutic agent, wherein said peptide and therapeutic may be separately or jointly administered, at the same or different times.

In one embodiment, the other therapeutic agent is an anti-cancer agent, an anti-viral or other anti-infectious agent, a cytokine or an immune agonist. Preferably, the other therapeutic agent is selected from CTLA-4-Ig, anti-PD-1, PD-L1 or PD-L2 fusion proteins, and EGFR antagonists.

Finally, the present invention also contemplates the use of an isolated VISTA antagonist in a
method for mapping the active site of VISTA, comprising: (a) incubating an isolated VISTA fusion protein with an isolated VISTA antagonist comprising a peptide that is identical to the amino acid sequence of SEQ ID NO:1 (Ser-Ser-Ala-Cys-Asp-Trp-Ile-Lys-Arg-Ser-Cys-His), or which comprises a peptide having an amino acid sequence which differs from SEQ ID NO:1 by at most 2 amino acid residues or a multimer, conjugate, or derivative thereof, wherein the derivative contains a modification of one or more amino acid residues or a linker group or other covalently linked group; and (b) determining the binding site of the isolated VISTA antagonist.

In one embodiment, the active site of VISTA binds to a VISTA receptor and mediates immune suppression.
In another embodiment, step (b) comprises domain deletion, domain swapping, amino acid mutagenesis, foot printing, NMR, X-ray crystallography or homology modeling.

### Brief Description of the Drawings

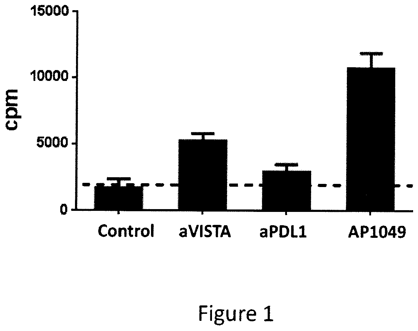

**Figure 1** shows that a VISTA antagonist peptide (SEQ ID NO:1) significantly enhances the proliferation of T cells as compared to an anti-VISTA antibody (aVISTA) and an anti-PD-Ll antibody (aPDL1). Myeloid CD11B+ APCs were incubated with OT2 CD4+ T cells, antigen, and a monoclonal antibody (aVISTA or aPDL1) or AP1049. Proliferation of T cells was measured by tritium incorporation at 72 hours.

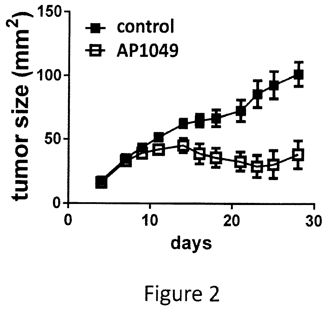

**Figure 2** shows that a VISTA antagonist peptide (SEQ ID NO:1) significantly enhances anti-tumor immunity. Female mice inoculated with MB49 tumors were treated with either PBS (control) or AP1049. Tumor size was measured by caliper every 2-3 days.

### Detailed Description of the Invention

In order that the invention herein described may be fully understood, the following detailed description is set forth. Various embodiments of the invention are described in detail and may be further illustrated by the provided examples.

Unless defined otherwise, all technical and scientific terms used herein have the same meaning as those commonly understood by one of ordinary skill in the art to which this invention belongs. Although methods and materials similar or equivalent to those described herein may be used in the invention or testing of the present invention, suitable methods and materials are described herein. The materials, methods and examples are illustrative only, and are not intended to be limiting.

### Definitions

As used in the description herein and throughout the claims that follow, the meaning of "a," "an," and "the" includes plural reference unless the context clearly dictates otherwise.

"Antagonist," as used herein, refers to a compound (preferably a peptide) that opposed the physiological effects of another compound. For example, at the receptor level, an antagonist is a compound that opposes the receptor-associated response normally induced by another agent that binds to and activates the biological activity the receptor. Likewise, at the ligand level, an antagonist is a compound that opposes the ligand-associated response normally induced when the ligand binds to its target receptor and/or accessory factors. In a specific embodiment, a VISTA antagonist is a compound, e.g., a peptide or derivative thereof, that binds to VISTA and opposes one or more of its biological activities, e.g., VISTA-mediated T cell suppression and/or VISTA-mediated suppression of anti-tumor immunity, thereby enhancing T cell-mediated immunity and/or anti-tumor immunity.

"Analog," as used herein, refers to a compound (preferably a peptide) whose structure is related to that of a given compound (preferably a peptide) but differs in chemical and biological properties.

"Antigen presenting cell," as used herein, refers broadly to professional antigen presenting cells (*e.g*., B lymphocytes, monocytes, dendritic cells, and Langerhans cells) as well as other antigen presenting cells (*e.g*., keratinocytes, endothelial cells, astrocytes, fibroblasts, and oligodendrocytes).

"Amino acid," as used herein refers broadly to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified (*e.g*., hydroxyproline, γ-carboxyglutamate, and O-phosphoserine.) Amino acid analogs refers to compounds that have the same basic chemical structure as a naturally occurring amino acid (*i.e*., a carbon that is bound to a hydrogen, a carboxyl group, an amino group), and an R group (*e.g*., homoserine, norleucine, methionine sulfoxide, methionine methyl sulfonium.) Analogs may have modified R groups (*e.g*., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

"Allergic disease," as used herein, refers broadly to a disease involving allergic reactions. More specifically, an "allergic disease" is defined as a disease for which an allergen is identified, where there is a strong correlation between exposure to that allergen and the onset of pathological change, and where that pathological change has been proven to have an immunological mechanism. Herein, an immunological mechanism means that leukocytes show an immune response to allergen stimulation.

"Autoimmune disease" as used herein, refers broadly to a disease or disorder arising from and directed against an individual's own tissues or a co-segregate or manifestation thereof or resulting condition therefrom.

"Cancer," as used herein, refers broadly to any neoplastic disease (whether invasive or metastatic) characterized by abnormal and uncontrolled cell division causing malignant growth or tumor (*e.g.*, unregulated cell growth.)

"Conservatively modified variants," as used herein, applies to both amino acid and nucleic acid sequences, and with respect to particular nucleic acid sequences, refers broadly to conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. "Silent variations" are one species of conservatively modified nucleic acid variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) may be modified to yield a functionally identical molecule.

"Costimulatory receptor," as used herein, refers broadly to receptors which transmit a costimulatory signal to an immune cell, *e.g.,* CD28 or ICOS.

"Cytoplasmic domain," as used herein, refers broadly to the portion of a protein which extends into the cytoplasm of a cell.

"Derivative" or "peptide derivative," as used herein, contain a modification of one or more amino acid residues or a linker group or other covalently linked group. Non-limiting examples of derivatives include N-acyl derivatives of the amino terminal or of another free amino group, esters of the carboxyl terminal or of another free carboxyl or hydroxy group, amides of the carboxyl terminal or of another free carboxyl group produced by reaction with ammonia or with a suitable amine, glycosylated derivatives, hydroxylated derivatives, nucleotidylated derivatives, ADP-ribosylated derivatives, pegylated derivatives, phosphorylated derivatives, derivatives conjugated to lipophilic moieties, and derivatives conjugated to an antibody or other biological ligand. Also included among the chemical derivatives are those obtained by modification of the peptide bond -CO-NH-, for example by reduction to -CH₂-NH- or alkylation to -CON(alkyl)-. Preferred derivatisation include, but are not limited tom C-terminal amidation and N-terminal acetylation, which removes the negative charge of the C terminus or removes the positive charge at the N-terminus, respectively. Blocking of the C- or N- terminus, such as by C-terminal amidation or N-terminal acetylation, may improve proteolytic stability due to reduced susceptibility to exoproteolytic digestion. Peptide derivatives having a C-terminal amide are represented with "NH₂" at the C-terminus.

"Diagnostic," as used herein, refers broadly to identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

"Diagnosing," as used herein refers broadly to classifying a disease or a symptom, determining a severity of the disease, monitoring disease progression, forecasting an outcome of a disease and/or prospects of recovery. The term "detecting" may also optionally encompass any of the foregoing. Diagnosis of a disease according to the present description may, in some examples, be affected by determining a level of a polynucleotide or a polypeptide of the present invention in a biological sample obtained from the subject, wherein the level determined can be correlated with predisposition to, or presence or absence of the disease. It should be noted that a "biological sample obtained from the subject" may also optionally comprise a sample that has not been physically removed from the subject.

"Effective amount," as used herein, refers broadly to the amount of a compound, antibody, antigen, or cells that, when administered to a patient for treating a disease, is sufficient to effect such treatment for the disease. The effective amount may be an amount effective for prophylaxis, and/or an amount effective for prevention. The effective amount may be an amount effective to reduce, an amount effective to prevent the incidence of signs/symptoms, to reduce the severity of the incidence of signs/symptoms, to eliminate the incidence of signs/symptoms, to slow the development of the incidence of signs/symptoms, to prevent the development of the incidence of signs/symptoms, and/or effect prophylaxis of the incidence of signs/symptoms. The "effective amount" may vary depending on the disease and its severity and the age, weight, medical history, susceptibility, and pre-existing conditions, of the patient to be treated. The term "effective amount" is synonymous with "therapeutically effective amount" for purposes of this invention.

"Extracellular domain," as used herein refers broadly to the portion of a protein that extend from the surface of a cell.

"Expression vector," as used herein, refers broadly to any recombinant expression system for the purpose of expressing a nucleic acid sequence of the invention *in vitro* or *in vivo,* constitutively or inducibly, in any cell, including prokaryotic, yeast, fungal, plant, insect or mammalian cell. The term includes linear or circular expression systems. The term includes expression systems that remain episomal or integrate into the host cell genome. The expression systems can have the ability to self-replicate or not, *i.e.,* drive only transient expression in a cell. The term includes recombinant expression cassettes which contain only the minimum elements needed for transcription of the recombinant nucleic acid.

"Homology," as used herein, refers broadly to a degree of similarity between a nucleic acid sequence and a reference nucleic acid sequence or between a polypeptide sequence and a reference polypeptide sequence. Homology may be partial or complete. Complete homology indicates that the nucleic acid or amino acid sequences are identical. A partially homologous nucleic acid or amino acid sequence is one that is not identical to the reference nucleic acid or amino acid sequence. The degree of homology can be determined by sequence comparison. The term "sequence identity" may be used interchangeably with "homology."

"Host cell," as used herein, refers broadly to refer to a cell into which a nucleic acid molecule of the invention, such as a recombinant expression vector of the invention, has been introduced. Host cells may be prokaryotic cells (*e.g*., *E. coli*), or eukaryotic cells such as yeast, insect (*e.g*., SF9), amphibian, or mammalian cells such as CHO, HeLa, HEK-293, *e.g.,* cultured cells, explants, and cells *in vivo.* The terms "host cell" and "recombinant host cell" are used interchangeably herein. It should be understood that such terms refer not only to the particular subject cell but to the progeny or potential progeny of such a cell. Because certain modifications may occur in succeeding generations due to either mutation or environmental influences, progeny may not, in fact, be identical to the parent cell, but are still included within the scope of the term as used herein.

"Immune response," as used herein, refers broadly to T cell-mediated and/or B cell-mediated immune responses that are influenced by modulation of T cell costimulation. Exemplary immune responses include B cell responses (*e.g*., antibody production) T cell responses (*e.g*., cytokine production, and cellular cytotoxicity) and activation of cytokine responsive cells, *e.g.,* macrophages. As used herein, the term "down modulation" with reference to the immune response includes a diminution in any one or more immune responses, while the term "up modulation" with reference to the immune response includes an increase in any one or more immune responses. It will be understood that up modulation of one type of immune response may lead to a corresponding downmodulation in another type of immune response. For example, up modulation of the production of certain cytokines (*e.g.*, IL-10) can lead to downmodulation of cellular immune responses.

"Inflammatory disease," as used herein, refers broadly to chronic or acute inflammatory diseases.

"Detectable label" as used herein, refers broadly to a composition detectable by spectroscopic, photochemical, biochemical, immunochemical, chemical, or other physical means.

"Mimetic" or "peptidomimetic," as used herein, refers to a fully or partially synthetic peptide that has the activity of a given peptide. Such a mimetic or peptidomimetic comprises one or more amino acid residues that is an artificial chemical mimetic of a corresponding naturally occurring amino acid, naturally occurring amino acid polymers and non-naturally occurring amino acid polymers.

Modifications of the VISTA and VISTA conjugate polypeptides described herein include, but are not limited to N-terminus modification, C-terminus modification, peptide bond modification (*e.g.*, CH₂-NH, CH₂-S, CH₂-S=O, O=C-NH, CH₂-O, CH₂-CH₂, S=C-NH, CH=CH or CF=CH), backbone modifications, and residue modification, *e.g*., by the addition of carbohydrate residues to form glycoproteins, by the addition of chemical residues such as PEG and/or XTEN, etc. Methods for preparing peptidomimetic compounds are well known in the art. Martin, (2010).

"Nucleic acid" or "nucleic acid sequence," as used herein, refers broadly to a deoxy-ribonucleotide or ribonucleotide oligonucleotide in either single- or doublestranded form. The term encompasses nucleic acids, *i.e.,* oligonucleotides, containing known analogs of natural nucleotides. The term also encompasses nucleic-acid-like structures with synthetic backbones. Unless otherwise indicated, a particular nucleic acid sequence also implicitly encompasses conservatively modified variants thereof (*e.g*., degenerate codon substitutions) and complementary sequences, as well as the sequence explicitly indicated. The term nucleic acid is used interchangeably with gene, cDNA, mRNA, oligonucleotide, and polynucleotide.

"Polypeptide," "peptide" and "protein," are used interchangeably and refer broadly to a polymer of amino acid residues. The terms apply to amino acid polymers in which one or more amino acid residue is an analog or mimetic of a corresponding naturally occurring amino acid, as well as to naturally occurring amino acid polymers. The terms apply to amino acid polymers in which one or more amino acid residue is an artificial chemical mimetic of a corresponding naturally occurring amino acid, as well as to naturally occurring amino acid polymers and non-naturally occurring amino acid polymer. Polypeptides can be modified, *e.g.,* by the addition of carbohydrate residues to form glycoproteins. The terms "polypeptide," "peptide" and "protein" include glycoproteins, as well as non-glycoproteins.

"Prophylactically effective amount," as used herein, refers broadly to the amount of a compound that, when administered to a patient for prophylaxis of a disease or prevention of the reoccurrence of a disease, is sufficient to effect such prophylaxis for the disease or reoccurrence. The prophylactically effective amount may be an amount effective to prevent the incidence of signs and/or symptoms. The "prophylactically effective amount" may vary depending on the disease and its severity and the age, weight, medical history, predisposition to conditions, preexisting conditions, of the patient to be treated.

"Prophylaxis," as used herein, refers broadly to a course of therapy where signs and/or symptoms are not present in the patient, are in remission, or were previously present in a patient. Prophylaxis includes preventing disease occurring subsequent to treatment of a disease in a patient. Further, prevention includes treating patients who may potentially develop the disease, especially patients who are susceptible to the disease (*e.g.,* members of a patent population, those with risk factors, or at risk for developing the disease).

"Recombinant" as used herein, refers broadly with reference to a product, *e.g.,* to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

"Sequence identity," as used herein, refers broadly to a degree of similarity between a nucleic acid sequence and a reference nucleic acid sequence or between a polypeptide sequence and a reference polypeptide sequence. Sequence identity (also synonymous with "homology") may be partial or complete. Complete sequence identity indicates that the nucleic acid or amino acid sequences are identical, i.e., 100% sequence identity. A partially homologous nucleic acid or amino acid sequence is one that is not identical to the reference nucleic acid or amino acid sequence. The degree of homology can be determined by sequence comparison, e.g., 60% identity, 70% identity, 80% identity, 90% identity, 95% identity, 97% identity, 98% identity, or 99% identity.

"Signs" of disease, as used herein, refers broadly to any abnormality indicative of disease, discoverable on examination of the patient; an objective indication of disease, in contrast to a symptom, which is a subjective indication of disease.

"Subject," as used herein, refers broadly to any animal that is in need of treatment either to alleviate a disease state or to prevent the occurrence or reoccurrence of a disease state. Also, "subject" as used herein, refers broadly to any animal that has risk factors, a history of disease, susceptibility, symptoms, and signs, was previously diagnosed, is at risk for, or is a member of a patient population for a disease. The subject may be a clinical patient such as a human or a veterinary patient such as a companion, domesticated, livestock, exotic, or zoo animal. The term "subject" may be used interchangeably with the term "patient."

"Symptoms" of disease as used herein, refers broadly to any morbid phenomenon or departure from the normal in structure, function, or sensation, experienced by the patient and indicative of disease.

"T cell," as used herein, refers broadly to CD4+ T cells and CD8+ T cells. The term T cell also includes both T helper 1 type T cells and T helper 2 type T cells.

"Therapy," "therapeutic," "treating," or "treatment", as used herein, refers broadly to treating a disease, arresting, or reducing the development of the disease or its clinical symptoms, and/or relieving the disease, causing regression of the disease or its clinical symptoms. Therapy encompasses prophylaxis, treatment, remedy, reduction, alleviation, and/or providing relief from a disease, signs, and/or symptoms of a disease. Therapy encompasses an alleviation of signs and/or symptoms in patients with ongoing disease signs and/or symptoms (*e.g.,* inflammation, pain). Therapy also encompasses "prophylaxis". The term "reduced", for purpose of therapy, refers broadly to the clinical significant reduction in signs and/or symptoms. Therapy includes treating relapses or recurrent signs and/or symptoms (*e.g.,* inflammation, pain). Therapy encompasses but is not limited to precluding the appearance of signs and/or symptoms anytime as well as reducing existing signs and/or symptoms and eliminating existing signs and/or symptoms. Therapy includes treating chronic disease ("maintenance") and acute disease. For example, treatment includes treating or preventing relapses or the recurrence of signs and/or symptoms (*e.g.,* inflammation, pain).

"Transmembrane domain," as used herein, refers broadly to an amino acid sequence of about 15 amino acid residues in length which spans the plasma membrane. More preferably, a transmembrane domain includes about at least 20, 25, 30, 35, 40, or 45 amino acid residues and spans the plasma membrane. Transmembrane domains are rich in hydrophobic residues, and typically have an alpha-helical structure. In an embodiment, at least 50%, 60%, 70%, 80%, 90%, 95% or more of the amino acids of a transmembrane domain are hydrophobic, *e.g.,* leucines, isoleucines, tyrosines, or tryptophans. Transmembrane domains are described in, for example, Zagotta, et al. (1996) Annu. Rev. Neurosci. 19:235-263.

"Tumor," as used herein, refers broadly to at least one cell or cell mass in the form of a tissue neoformation, in particular in the form of a spontaneous, autonomous and irreversible excess growth, which is more or less disinhibited, of endogenous tissue, which growth is as a rule associated with the more or less pronounced loss of specific cell and tissue functions. This cell or cell mass is not effectively inhibited, in regard to its growth, by itself or by the regulatory mechanisms of the host organism, *e.g.,* melanoma or carcinoma. Tumor antigens not only include antigens present in or on the malignant cells themselves, but also include antigens present on the stromal supporting tissue of tumors including endothelial cells and other blood vessel components.

"Vector," as used herein, refers broadly to a nucleic acid molecule capable of transporting another nucleic acid molecule to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.,* bacterial vectors having a bacterial origin of replication and episomal mammalian vectors). Other vectors (*e.g.,* non-episomal mammalian vectors) are integrated into the genome of a host cell upon introduction into the host cell, and thereby are replicated along with the host genome. Moreover, certain vectors are capable of directing the expression of genes to which they are operatively linked. Vectors are referred to herein as "recombinant expression vectors" or simply "expression vectors". In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (*e.g.,* replication defective retroviruses, adenoviruses and adeno-associated viruses), which serve equivalent functions. The techniques and procedures are generally performed according to conventional methods well known in the art and as described in various general and more specific references that are cited and discussed throughout the present specification. *See, e.g*., Sambrook, et al. (2001) Molec. Cloning: Lab. Manual [3rd Ed] Cold Spring Harbor Laboratory Press. Standard techniques may be used for recombinant DNA, oligonucleotide synthesis, and tissue culture, and transformation (*e.g.,* electroporation, lipofection). Enzymatic reactions and purification techniques may be performed according to manufacturer's specifications or as commonly accomplished in the art or as described herein.

The nomenclatures utilized in connection with, and the laboratory procedures and techniques of, analytical chemistry, synthetic organic chemistry, and medicinal and pharmaceutical chemistry described herein are those well known and commonly used in the art. Standard techniques may be used for chemical syntheses, chemical analyses, pharmaceutical preparation, formulation, and delivery, and treatment of patients.

### VISTA and VISTA Antagonists

This application relates to a peptide antagonist that can recognize and suppress the inhibitory activity of VISTA. This peptide, designated herein as AP1049, was discovered through phage display and shown to exhibit superior bioactivity when compared to an anti-VISTA monoclonal antibody. Given its neutralizing activity, AP1049 can be used to, e.g., treat cancer and/or pathogenic, i.e., bacterial, fungal, parasite or viral infections and enhance anti-tumor immune responses and suppress tumor growth.

Accordingly, the present invention is a VISTA antagonistic peptide, as well as multimers, conjugates, and derivatives thereof and methods of using this peptide to inhibit or suppress the activity of VISTA. According to the present invention the derivative thereof is one which contains a modification of one or more amino acid residues or a linker group or other covalently linked group. As used herein, the term "peptide" denotes an amino acid polymer that is composed of at least two amino acids covalently linked by an amide bond. Peptides of the present invention are desirably 10 to 20 residues in length, or more desirably 12 to 15 residues in length. In certain embodiments, a VISTA antagonistic peptide is a 12 to 20 residue peptide containing the amino acid sequence of SEQ ID NO:1. In other embodiments of the present invention, the isolated VISTA antagonist comprises a peptide that is identical to the amino acid sequence of SEQ ID NO:1 (Ser-Ser-Ala-Cys-Asp-Trp-Ile-Lys-Arg-Ser-Cys-His), or which comprises a peptide having an amino acid sequence that differs from SEQ ID NO:1 by at most 1 amino acid residue or at most 2 amino acid residues, or a multimer, conjugate, or derivative. In yet other embodiments of the invention, the isolated VISTA antagonist consists of the amino acid sequence of SEQ ID NO:1 (Ser-Ser-Ala-Cys-Asp-Trp-Ile-Lys-Arg-Ser-Cys-His).

In certain embodiments of the present invention, cysteine residues at positions 4 and 11 of the VISTA antagonistic peptide (or their corresponding positions in a variant of the VISTA antagonist) form a disulfide bridge.

In accordance with the present invention, multimers, conjugates, and derivatives of the peptide of the invention are also provided.

An analog is a molecule that differs in chemical structure from a parent compound, for example a homolog (differing by an increment in the chemical structure, such as a difference one amino acid residue), a structure that differs by one or more functional groups, or a change in ionization. Structural analogs are often found using quantitative structure activity relationships (QSAR), with techniques such as those disclosed in Remington (The Science and Practice of Pharmacology, 19th Edition (1995), chapter 28).

Analogs can be prepared by modifying the amino acids sequence of SEQ ID NO:1. The simplest modifications involve the substitution of one or more amino acids for amino acids having similar physiochemical and/or structural properties. These so-called conservative substitutions are likely to have minimal impact on the activity and/or structure of the resultant peptide. Examples of conservative substitutions include substituting a serine with a threonine, substituting alanine with a serine or valine, substituting aspartic acid with glutamic acid, substituting tryptophan with a tyrosine, substituting isoleucine with leucine or valine, substituting arginine with lysine, and/or substituting histidine with arginine or lysine. Conservative substitutions generally maintain (a) the structure of the peptide backbone in the area of the substitution, for example, as a helical conformation, (b) the charge or hydrophobicity of the molecule at the target site, or (c) the bulk of the side chain.

Amino acid substitutions are typically classified in one or more categories, including polar, hydrophobic, acidic, basic and aromatic, according to their side chains. Examples of polar amino acids include those having side chain functional groups such as hydroxyl, sulfhydryl, and amide, as well as the acidic and basic amino acids. Polar amino acids include, without limitation, asparagine, cysteine, glutamine, histidine, selenocysteine, serine, threonine, tryptophan and tyrosine. Examples of hydrophobic or non-polar amino acids include those residues having non-polar aliphatic side chains, such as, without limitation, leucine, isoleucine, valine, glycine, alanine, proline, methionine and phenylalanine. Examples of basic amino acid residues include those having a basic side chain, such as an amino or guanidino group. Basic amino acid residues include, without limitation, arginine, homolysine and lysine. Examples of acidic amino acid residues include those having an acidic side chain functional group, such as a carboxy group. Acidic amino acid residues include, without limitation aspartic acid and glutamic acid. Aromatic amino acids include those having an aromatic side chain group. Examples of aromatic amino acids include, without limitation, biphenylalanine, histidine, 2-napthylalananine, pentafluorophenylalanine, phenylalanine, tryptophan and tyrosine. It is noted that some amino acids are classified in more than one group, for example, histidine, tryptophan and tyrosine are classified as both polar and aromatic amino acids. Additional amino acids that are classified in each of the above groups are known to those of ordinary skill in the art.

As used herein, a peptide derivative is a molecule which retains the primary amino acids of the peptide, however, the N-terminus, C-terminus, and/or one or more of the side chains of the amino acids therein have been chemically altered or derivatized. Such derivatized peptides include, for example, naturally occurring amino acid derivatives, for example, 4-hydroxyproline for proline, 5-hydroxylysine for lysine, homoserine for serine, ornithine for lysine, and the like. Other derivatives or modifications include, *e.g.,* a label, such as fluorescein or tetramethylrhodamine; or one or more post-translational modifications such as acetylation, amidation, formylation, hydroxylation, methylation, phosphorylation, sulfatation, glycosylation, or lipidation. Indeed, certain chemical modifications, in particular N-terminal glycosylation, have been shown to increase the stability of peptides in human serum (Powell et al. (1993) Pharma. Res. 10:1268-1273). Peptide derivatives also include those with increased membrane permeability obtained by N-myristoylation (Brand, et al. (1996) Am. J. Physiol. Cell. Physiol. 270:C1362-C1369).

In addition, a peptide derivative of the invention can include a cell-penetrating sequence which facilitates, enhances, or increases the transmembrane transport or intracellular delivery of the peptide into a cell. For example, a variety of proteins, including the HIV-1 Tat transcription factor, *Drosophila* Antennapedia transcription factor, as well as the herpes simplex virus VP22 protein have been shown to facilitate transport of proteins into the cell (Wadia and Dowdy (2002) Curr. Opin. Biotechnol. 13:52-56). Further, an arginine-rich peptide (Futaki (2002) Int. J. Pharm. 245:1-7), a polylysine peptide containing Tat PTD (Hashida, et al. (2004) Br. J. Cancer 90(6):1252-8), Pep-1 (Deshayes, et al. (2004) Biochemistry 43(6) :1449-57) or an HSP70 protein or fragment thereof (WO 00/31113) is suitable for enhancing intracellular delivery of a peptide of the invention into the cell.

While a peptide of the invention can be derivatized with by one of the above indicated modifications, it is understood that a peptide of this invention may contain more than one of the above described modifications within the same peptide.

A mimetic or peptidomimetic according to the description refers to a synthetic chemical compound which has substantially the same structural and/or functional characteristics of a peptide of the invention. The mimetic can be entirely composed of synthetic, non-natural amino acid analogues, or can be a chimeric molecule including one or more natural peptide amino acids and one or more non-natural amino acid analogs. The mimetic can also incorporate any number of natural amino acid conservative substitutions as long as such substitutions do not destroy the activity of the mimetic. Routine testing can be used to determine whether a mimetic has the requisite activity, *e.g.,* that it can inhibit the activity of VISTA.

The phrase "substantially the same," when used in reference to a mimetic or peptidomimetic, means that the mimetic or peptidomimetic has one or more activities or functions of the referenced molecule, *e.g.,* the ability to enhance T cell proliferation.

There are clear advantages for using a mimetic of a given peptide. For example, there are considerable cost savings and improved patient compliance associated with peptidomimetics, since they can be administered orally compared with parenteral administration for peptides. Furthermore, peptidomimetics are much cheaper to produce than peptides.

Thus, a peptide of this invention has utility in the development of such small chemical compounds with similar biological activities and therefore with similar therapeutic utilities. The techniques of developing peptidomimetics are conventional. For example, peptide bonds can be replaced by non-peptide bonds or non-natural amino acids that allow the peptidomimetic to adopt a similar structure, and therefore biological activity, to the original peptide. Further modifications can also be made by replacing chemical groups of the amino acids with other chemical groups of similar structure. The development of peptidomimetics can be aided by determining the tertiary structure of the original peptide by NMR spectroscopy, crystallography and/or computer-aided molecular modeling. These techniques aid in the development of novel compositions of higher potency and/or greater bioavailability and/or greater stability than the original peptide (Dean (1994) BioEssays 16:683-687; Cohen & Shatzmiller (1993) J. Mol. Graph. 11:166-173; Wiley & Rich (1993) Med. Res. Rev. 13:327-384; Moore (1994) Trends Pharmacol. Sci. 15:124-129; Hruby (1993) Biopolymers 33:1073-1082; Bugg, et al. (1993) Sci. Am. 269:92-98). Once a potential peptidomimetic compound is identified, it may be synthesized and assayed using an assay described herein or any other appropriate assay for monitoring VISTA activity.

Peptide mimetic compositions can contain any combination of non-natural structural components, which are typically from three structural groups: residue linkage groups other than the natural amide bond ("peptide bond") linkages; non-natural residues in place of naturally occurring amino acid residues; residues which induce secondary structural mimicry, *i.e.,* induce or stabilize a secondary structure, *e.g.,* a beta turn, gamma turn, beta sheet, alpha helix conformation, and the like; or other changes which confer resistance to proteolysis. For example, a peptide can be characterized as a mimetic when one or more of the residues are joined by chemical means other than an amide bond. Individual peptidomimetic residues can be joined by amide bonds, non-natural and non-amide chemical bonds other chemical bonds or coupling means including, for example, glutaraldehyde, N-hydroxysuccinimide esters, bifunctional maleimides, N,N'-dicyclohexylcarbodiimide (DCC) or N,N'-diisopropyl-carbodiimide (DIC). Linking groups alternative to the amide bond include, for example, ketomethylene (*e.g.,-*C(=O)-CH₂- for -C(=O)-NH-), aminomethylene (CH₂-NH), ethylene, olefin (CH=CH), ether (CH₂-O), thioether (CH₂-S), tetrazole (CN₄-), thiazole, retroamide, thioamide, or ester (see, *e.g.,* Spatola (1983) in Chemistry and Biochemistry of Amino Acids, Peptides and Proteins, 7:267-357, "Peptide and Backbone Modifications," Marcel Decker, NY).

As discussed, a peptide can be characterized as a mimetic by containing one or more non-natural residues in place of a naturally occurring amino acid residue. Non-natural residues are known in the art. Particular non-limiting examples of non-natural residues useful as mimetics of natural amino acid residues are mimetics of aromatic amino acids include, for example, D- or L-naphylalanine; D- or L-phenylglycine; D- or L-2 thieneylalanine; D- or L-1, -2, 3-, or 4-pyreneylalanine; D- or L-3 thieneylalanine; D- or L-(2-pyridinyl)-alanine; D- or L-(3-pyridinyl)-alanine; D- or L-(2-pyrazinyl)-alanine; D- or L-(4-isopropyl)-phenylglycine; D-(trifluoromethyl)-phenylglycine; D-(trifluoromethyl)-phenylalanine; D-p-fluoro-phenylalanine; D- or L-p-biphenylphenylalanine; D- or L-p-methoxy-biphenyl-phenylalanine; and D- or L-2-indole(alkyl)alanines, where alkyl can be substituted or unsubstituted methyl, ethyl, propyl, hexyl, butyl, pentyl, isopropyl, iso-butyl, sec-isotyl, iso-pentyl, or a non-acidic amino acid. Aromatic rings of a non-natural amino acid that can be used in place a natural aromatic ring include, for example, thiazolyl, thiophenyl, pyrazolyl, benzimidazolyl, naphthyl, furanyl, pyrrolyl, and pyridyl aromatic rings.

Mimetics of acidic amino acids can be generated by substitution with non-carboxylate amino acids while maintaining a negative charge; (phosphono)alanine; and sulfated threonine. Carboxyl side groups (*e.g.,* aspartyl or glutamyl) can also be selectively modified by reaction with carbodiimides (R'-N-C-N-R') including, for example, 1-cyclohexyl-3(2-morpholinyl-(4-ethyl)carbodiimide or 1-ethyl-3(4-azonia-4,4-dimetholpentyl)carbodiimide. Aspartyl or glutamyl groups can also be converted to asparaginyl and glutaminyl groups by reaction with ammonium ions.

Lysine mimetics can be generated (and amino terminal residues can be altered) by reacting lysinyl with succinic or other carboxylic acid anhydrides. Lysine and other alpha-amino-containing residue mimetics can also be generated by reaction with imidoesters, such as methyl picolinimidate, pyridoxal phosphate, pyridoxal, chloroborohydride, trinitrobenzenesulfonic acid, O-methylisourea, 2,4, pentanedione, and transamidase-catalyzed reactions with glyoxylate.

One or more residues can also be replaced by an amino acid (or peptidomimetic residue) of the opposite chirality. Thus, any amino acid naturally occurring in the L-configuration (which can also be referred to as R or S, depending upon the structure of the chemical entity) can be replaced with the same amino acid or a mimetic, but of the opposite chirality, referred to as the D-amino acid, but which can additionally be referred to as the R- or S-form.

As will be appreciated by one skilled in the art, a peptidomimetic of the present description can also include one or more of the modifications described herein for derivatized peptides, *e.g.,* a detectable label (such as an effector label or a radionuclide), a therapeutic agent (such as a chemotherapeutic agent), one or more post-translational modifications, or cell-penetrating sequence.

For example, the VISTA antagonists described herein may be modified post-translationally to add effector labels such as chemical linkers, detectable labels such as for example fluorescent dyes, enzymes, substrates, bioluminescent materials, radioactive materials, and chemiluminescent labels, or functional labels such as for example streptavidin, avidin, biotin, a cytotoxin, a cytotoxic agent, and radioactive materials. Further exemplary enzymes include, but are not limited to, horseradish peroxidase, acetylcholinesterase, alkaline phosphatase, β-galactosidase and luciferase. Further exemplary fluorescent materials include, but are not limited to, rhodamine, fluorescein, fluorescein isothiocyanate, umbelliferone, dichlorotriazinylamine, phycoerythrin and dansyl chloride. Further exemplary chemiluminescent labels include, but are not limited to, luminol. Further exemplary bioluminescent materials include, but are not limited to, luciferin, luciferase, and aequorin. Further exemplary radioactive materials include, but are not limited to, bismuth-213 (²¹³Bs), carbon-14 (¹⁴C), carbon-11 (¹¹C), chlorine-18 (Cl¹⁸), chromium-51 (⁵¹Cr), cobalt-57 (⁵⁷Co), cobalt-60 (⁶⁰Co), copper-64 (⁶⁴Cu), copper-67 (⁶⁷Cu), dysprosium-165 (¹⁶⁵Dy), erbium-169 (¹⁶⁹Er), fluorine-18 (¹⁸F), gallium-67 (⁶⁷Ga), gallium-68 (⁶⁸Ga), germanium-68 (⁶⁸Ge), holmium-166 (¹⁶⁶Ho), indium-111 (¹¹¹In), iodine-125 (¹²⁵I), iodine-123 (¹²⁴I), iodine-124 (¹²⁴I), iodine-131 (¹³¹I), iridium-192 (¹⁹²Ir),iron-59 (⁵⁹Fe), krypton-81 (⁸¹Kr), lead-212 (²¹²Pb), lutetium-177 (¹⁷⁷Lu), molybdenum-99 (⁹⁹Mo), nitrogen-13 (¹³N), oxygen-15 (¹⁵O), palladium-103 (¹⁰³Pd), phosphorus-32 (³²P), potassium-42 (⁴²K), rhenium-186 (¹⁸⁶Re), rhenium-188 (¹⁸⁸Re), rubidium-81 (⁸¹Rb), rubidium-82 (⁸²Rb), samarium-153 (¹⁵³Sm), selenium-75 (⁷⁵Se), sodium-24 (²⁴Na), strontium-82 (⁸²Sr), strontium-89 (⁸⁹Sr), sulfur 35 (³⁵S), technetium-99m (⁹⁹Tc), thallium-201 (²⁰¹Tl), tritium (³H), xenon-133 (¹³³Xe), ytterbium-169 (¹⁶⁹Yb), ytterbium-177 (¹⁷⁷Yb), and yttrium-90 (⁹⁰Y).

Additionally, the VISTA antagonists provided herein may be modified to add a therapeutic agent including, but not limited to, chemotherapeutic agents such as carboplatin, cisplatin, paclitaxel, gemcitabine, calicheamicin, doxorubicin, 5-fluorouracil, mitomycin C, actinomycin D, cyclophosphamide, vincristine, bleomycin, VEGF antagonists, EGFR antagonists, platins, taxols, irinotecan, 5-fluorouracil, gemcytabine, leucovorine, steroids, cyclophosphamide, melphalan, vinca alkaloids (*e.g.,* vinblastine, vincristine, vindesine and vinorelbine), mustines, tyrosine kinase inhibitors, radiotherapy, sex hormone antagonists, selective androgen receptor modulators, selective estrogen receptor modulators, PDGF antagonists, TNF antagonists, IL-1 antagonists, interleukins (*e.g.,* IL-12 or IL-2), IL-12R antagonists, Toxin conjugated monoclonal antibodies, tumor antigen specific monoclonal antibodies, Erbitux®, Avastin®, Pertuzumab, anti-CD20 antibodies, Rituxan®, ocrelizumab, ofatumumab, DXL625, Herceptin®, or any combination thereof. Toxic enzymes from plants and bacteria such as ricin, diphtheria toxin and *Pseudomonas* toxin may be conjugated to the VISTA antagonists to generate cell-type-specific-killing reagents. Youle, et al. (1980) Proc. Nat'l Acad. Sci. USA 77: 5483; Gilliland, et al. (1980) Proc. Nat'l Acad. Sci. USA 77: 4539; Krolick, et al. (1980) Proc. Nat'l Acad. Sci. USA 77: 5419.

Furthermore, the VISTA antagonists described herein may be conjugated to a radionuclide that emits alpha or beta particles (*e.g.,* radioimmunoconjuagtes). Such radioactive isotopes include but are not limited to beta-emitters such as phosphorus-32 (³²P), scandium-47 (⁴⁷Sc), copper-67 (¹⁷Cu), gallium-67 (⁶⁷Ga), yttrium-88 (⁸⁸Y), yttrium-90 (⁹⁰Y), iodine-125 (¹²⁵I), iodine-131 (¹³¹I), samarium-153 (¹⁵³Sm), lutetium-177 (¹⁷⁷Lu), rhenium-186 (¹⁸⁶Re), rhenium-188 (¹⁸⁸Re), and alpha-emitters such as astatine-211 (²¹¹At), lead-212 (²¹²Pb), bismuth-212 (²¹²Bi), bismuth-213 (²¹³Bi) or actinium-225 (¹²⁵Ac).

Methods are known in the art for conjugating a VISTA antagonist described herein to a label, such as those methods described by Hunter, et al (1962) Nature 144: 945; David, et al. (1974) Biochemistry 13: 1014; Pain, et al. (1981) J. Immunol. Meth. 40: 219; and Nygren (1982) Histochem and Cytochem, 30: 407.

Additionally, the VISTA antagonists described herein may comprise another moiety, i.e., a "targeting moiety," that targets the antagonist peptide to a target site (such as a cancer cell, a tumor, a virally-infected cell, etc). The targeting moiety may be selected from an antibody or ligand that binds to an antigen, a receptor expressed by the target cell or an infectious agent.

The VISTA antagonist (as well as multimers, conjugates, analogs, derivatives and mimetics thereof) may also be directly or indirectly attached to an immunoglobulin polypeptide or a fragment thereof, e.g., a antibody constant region.

A "conjugate," as used herein, refers to a compound having at least one isolated VISTA antagonist peptide and one immunoglobulin polypeptide or a fragment thereof, e.g., antibody constant region, joined at the polypeptide level, with or without the use of a linker. A conjugate may be a fusion polypeptide produced as the result of joining at the nucleic acid level of genes encoding at least one natriuretic peptide and one antibody constant region, with or without a coding sequence for a peptide linker.

Such VISTA antagonist peptide-antibody conjugates may have a higher serum stability, e.g., at least 20%, preferably at least 30%, 50%, 80%, 100%, 200% or more, increase in the serum half-life when compared with the antagonist peptide without the antibody constant region under the same conditions. A human antibody, e.g., a human IgG, such as IgG1, IgG2, IgG3 or IgG4, is frequently used to derive a constant region or a fragment thereof for the purpose of making a natriuretic peptide conjugate of this invention.

As used herein, an "antibody (or immunoglobulin) constant region" refers to a polypeptide that corresponds to at least a portion of the constant region of an antibody heavy chain or light chain, such portion including at least one constant domain (e.g., the constant domain of CL or one of the constant domains of C_{H}). For example, an "antibody constant region" used for making the conjugates of this invention may be derived from an antibody heavy chain and include two out of three (C_{H}2 and C_{H}3 for IgA, IgD, and IgG) or three out of four (C_{H}2, CH3, and CH4, for IgE and IgM) constant domains; the first constant domain (C_{H}I) may be present in some cases but may be excluded in others. Such an antibody constant region can be obtained by a variety of means, e.g., by a recombinant method or synthetic method, or by purification subsequent to enzymatic digestion, for instance, pepsin or papain digestion of an intact antibody or an antibody heavy or light chain.

Further encompassed by this term as used in this application are polypeptides having a substantial sequence identity (for instance, at least 80%, 85%, 90%, 95% or more) to the corresponding amino acid sequence of an antibody heavy or light chain constant region or a portion thereof that contains at least one constant domain nearest to the C-terminus of the antibody chain, so long as the presence of such an "antibody constant region" in a VISTA antagonist peptide-antibody constant region conjugate renders the conjugate a higher serum stability.

Additionally, the peptide, multimer, conjugate, analog, derivative or mimetic may be modified to increase certain properties, e.g., biological half life. Various approaches are possible including, but not limited to, N-terminal modification/conjugation (e.g., lipidation or acetylation), C-terminal modification/conjugation (e.g., lipidation or acetylation), amino acid substitutions (i.e., substitution of natural amino acid with unnatural amino acids, such as D-conformation, N-methylation, tetra-substitution, beta-amino acids, etc.), peptide backbone modifications (e.g., chemical modification of peptide bonds, such as simple reductions or replacement of carbonyl or amide groups with esters, sulfides and alkyls), side chain modifications and/or cyclization (e.g., disulfide bond formation).

In one embodiment, the peptide may be pegylated to, e.g., increase the biological (*e.g.,* serum) half life of the antibody. To pegylate a peptide, typically it is reacted with polyethylene glycol (PEG), such as a reactive ester or aldehyde derivative of PEG, under conditions in which one or more PEG groups become attached to the peptide. Preferably, the pegylation is carried out via an acylation reaction or an alkylation reaction with a reactive PEG molecule (or an analogous reactive water-soluble polymer.

Similarly, in another embodiment, a peptide, multimer, conjugate, or derivative may be modified by conjugation of polysialic acid (PSA) to increase half-life.

Additionally, the peptide, multimer, conjugate, analog, derivative or mimetic may be modified, e.g., genetically fused or chemically conjugated, to comprise extended recombinant polypeptide (XTEN), through a process called XTENylation, to improve its half life. XTEN is a long, hydrophilic, and unstructured protein-based polymer of 864 amino acids. See, e.g., WO 2013/130683.
When attached to a molecule of interest, greatly increases the effective size of the molecule, thereby prolonging its presence in serum by slowing kidney clearance in a manner analogous to that of PEG. In addition to slowing kidney clearance, attachment to XTEN can also inhibit receptor-mediated clearance by reducing the ligand's affinity for its receptor. XTEN coupling chemistries include, but are not limited to, Thiol-XTEN; Maleimide-XTEN; Alkyne-XTEN; and Iodoacetyl-XTEN.

Moreover, the peptide, multimer, conjugate, analog, derivative or mimetic may be modified with recombinant albumin, e.g., Novozymes Recombumin®, to improve half life. The peptide can be genetically fused or chemically conjugated to a recombinant albumin using standard protocols.

Furthermore, the peptide, multimer, conjugate, analog, derivative or mimetic may be modified by the addition and/or removal of specific amino acids to and/or from the peptide. For example, a number of specific amino acids may be added to the peptide, thereby strengthening or tightening its molecular structure to make it less susceptible to biological degradation and, thus, providing a longer life-span in the blood, using, e.g., Zealand Structure Induced Probe (SIP®) tail technology.

Yet another exemplary method for improving the stability and therapeutic potential of peptides, analogs, derivatives or mimetics is multimerization. For example, a multimer may comprise two or more copies, e.g., 2, 3, 4, 5, 6, 7 or more, of the isolated VISTA antagonist or variant thereof. Multimers include both homomultimers and heteromultimers. Multimerization can result in increased peptide stability, higher binding strength (due to multiple valencies in the molecule), and/or improved pharmacokinetic properties.

Another exemplary approach for improving the stability and, thus, therapeutic potential of the VISTA antagonist peptides, multimers, conjugates, analogs, derivatives or mimetics disclosed herein is the addition of acetyl groups to the N and/or C terminus of the peptide. Acetylation may protect the peptide from exopeptidases, thereby extending the half-life of the peptide.

### Production of VISTA Antagonists

The peptide multimer, conjugate, analog, derivative or mimetic can be produced and isolated using any method known in the art. Peptides can be synthesized, whole or in part, using chemical methods known in the art (see, *e.g.,* Caruthers (1980) Nucleic Acids Res. Symp. Ser. 215-223; Horn (1980) Nucleic Acids Res. Symp. Ser. 225-232; and Banga (1995) Therapeutic Peptides and Proteins, Formulation, Processing and Delivery Systems, Technomic Publishing Co., Lancaster, Pa.). Peptide synthesis can be performed using various solid-phase techniques (see, *e.g.,* Roberge (1995) Science 269:202; Merrifield (1997) Methods Enzymol. 289:3-13) and automated synthesis may be achieved, *e.g.,* using the ABI 431A Peptide Synthesizer (Perkin Elmer) in accordance with the manufacturer's instructions.

Individual synthetic residues and peptides incorporating mimetics can be synthesized using a variety of procedures and methodologies known in the art (see, *e.g.*, Organic Syntheses Collective Volumes, Gilman, et al. (Eds) John Wiley & Sons, Inc., NY). Peptides and peptide mimetics can also be synthesized using combinatorial methodologies. Techniques for generating peptide and peptidomimetic libraries are well-known, and include, for example, multipin, tea bag, and split-couple-mix techniques (see, for example, al-Obeidi (1998) Mol. Biotechnol. 9:205-223; Hruby (1997) Curr. Opin. Chem. Biol. 1:114-119; Ostergaard (1997) Mol. Divers. 3:17-27; and Ostresh (1996) Methods Enzymol. 267:220-234). Modified peptides can be further produced by chemical modification methods (see, for example, Belousov (1997) Nucleic Acids Res. 25:3440-3444; Frenkel (1995) Free Radic. Biol. Med. 19:373-380; and Blommers (1994) Biochemistry 33:7886-7896).

Alternatively, a peptide of this invention can be prepared in recombinant protein systems using polynucleotide sequences encoding the peptides. By way of illustration, a nucleic acid molecule encoding a peptide of the invention is introduced into a host cell, such as bacteria, yeast or mammalian cell, under conditions suitable for expression of the peptide, and the peptide is purified or isolated using methods known in the art. See, *e.g*., Deutscher et al. (1990) Guide to Protein Purification: Methods in Enzymology Vol. 182, Academic Press. In particular embodiments, the peptide, or derivative thereof is isolated and/or purified to homogeneity (*e.g.* greater than 90% purity).

It is contemplated that the peptide disclosed herein can be used as a lead compound for the design and synthesis of compounds with improved efficacy, clearance, half-lives, and the like.

One approach includes structure-activity relationship (SAR) analysis (*e.g.,* NMR analysis) to determine specific binding interactions between the peptide and VISTA to facilitate the development of more efficacious agents. Agents identified in such SAR analysis or from agent libraries can then be screened for their ability to, *e.g.,* decrease the activity of VISTA and/or enhance T cell proliferation.

### Pharmaceutical Compositions

The VISTA antagonist peptide, multimer, conjugate, analog, derivative and mimetic thereof described herein can be provided in a pharmaceutical composition.

A "pharmaceutical composition" refers to a chemical or biological composition suitable for administration to a mammal. Such compositions may be specifically formulated for administration *via* one or more of a number of routes, including but not limited to buccal, epicutaneous, epidural, inhalation, intraarterial, intracardial, intracerebroventricular, intradermal, intramuscular, intranasal, intraocular, intraperitoneal, intraspinal, intrathecal, intravenous, oral, parenteral, rectally via an enema or suppository, subcutaneous, subdermal, sublingual, transdermal, and transmucosal. In addition, administration may occur by means of injection, powder, liquid, gel, drops, or other means of administration.

A "pharmaceutical excipient" or a "pharmaceutically acceptable excipient" is a carrier, usually a liquid, in which an active therapeutic agent is formulated. In one embodiment of the invention, the active therapeutic agent is a humanized antibody described herein, or one or more fragments thereof. The excipient generally does not provide any pharmacological activity to the formulation, though it may provide chemical and/or biological stability, and release characteristics. Exemplary formulations may be found, for example, in Grennaro (2005) [Ed.] Remington: The Science and Practice of Pharmacy [21st Ed.]

Pharmaceutical compositions typically must be sterile and stable under the conditions of manufacture and storage. The invention contemplates that the pharmaceutical composition is present in lyophilized form. The composition may be formulated as a solution, microemulsion, liposome, or other ordered structure suitable to high drug concentration. The carrier may be a solvent or dispersion medium containing, for example, water, ethanol, polyol (for example, glycerol, propylene glycol, and liquid polyethylene glycol), and suitable mixtures thereof. The invention further contemplates the inclusion of a stabilizer in the pharmaceutical composition.

The VISTA antagonist peptide, multimer, conjugate, analog, derivative and mimetic thereof described herein may be formulated into pharmaceutical compositions of various dosage forms. To prepare the pharmaceutical compositions of the invention, at least one VISTA antagonist as the active ingredient may be intimately mixed with appropriate carriers and additives according to techniques well known to those skilled in the art of pharmaceutical formulations. *See* Grennaro (2005) [Ed.] Remington: The Science and Practice of Pharmacy [21st Ed.] For example, the antagonists described herein may be formulated in phosphate buffered saline pH 7.2 and supplied as a 5.0 mg/mL clear colorless liquid solution.

Similarly, compositions for liquid preparations include solutions, emulsions, dispersions, suspensions, syrups, and elixirs, with suitable carriers and additives including but not limited to water, alcohols, oils, glycols, preservatives, flavoring agents, coloring agents, and suspending agents. Typical preparations for parenteral administration comprise the active ingredient with a carrier such as sterile water or parenterally acceptable oil including but not limited to polyethylene glycol, polyvinyl pyrrolidone, lecithin, arachis oil or sesame oil, with other additives for aiding solubility or preservation may also be included. In the case of a solution, it may be lyophilized to a powder and then reconstituted immediately prior to use. For dispersions and suspensions, appropriate carriers and additives include aqueous gums, celluloses, silicates, or oils.

For each of the recited embodiments, the VISTA antagonist peptides, multimers, conjugates, and derivatives thereof may be administered by a variety of dosage forms. Any biologically-acceptable dosage form known to persons of ordinary skill in the art, and combinations thereof, are contemplated. Examples of such dosage forms include, without limitation, reconstitutable powders, elixirs, liquids, solutions, suspensions, emulsions, powders, granules, particles, microparticles, dispersible granules, cachets, inhalants, aerosol inhalants, patches, particle inhalants, implants, depot implants, injectables (including subcutaneous, intramuscular, intravenous, and intradermal), infusions, and combinations thereof.

In many cases, it will be preferable to include isotonic agents, *e.g.,* sugars, polyalcohols such as mannitol, sorbitol, or sodium chloride in the composition. Prolonged absorption of the injectable compositions may be brought about by including in the composition an agent which delays absorption, *e.g.,* monostearate salts and gelatin. Moreover, the compounds described herein may be formulated in a time release formulation, *e.g.* in a composition that includes a slow release polymer. The VISTA and VISTA conjugate may be prepared with carriers that will protect the compound against rapid release, such as a controlled release formulation, including implants and microencapsulated delivery systems. Biodegradable, biocompatible polymers may be used, such as ethylene vinyl acetate, polyanhydrides, polyglycolic acid, collagen, polyorthoesters, polylactic acid and polylactic, polyglycolic copolymers (PLG). Many methods for the preparation of such formulations are known to those skilled in the art.

A person of skill in the art would be able to determine an effective dosage and frequency of administration through routine experimentation, for example guided by the disclosure herein and the teachings in Goodman, et al. (2011) Goodman & Gilman's The Pharmacological Basis of Therapeutics [12th Ed.]; Howland, et al. (2005) Lippincott's Illustrated Reviews: Pharmacology [2nd Ed.]; and Golan, (2008) Principles of Pharmacology: The Pathophysiologic Basis of Drug Therapy [2nd Ed.] *See, also,* Grennaro (2005) [Ed.] Remington: The Science and Practice of Pharmacy [21st Ed.]

The compositions described herein may be administered in any of the following routes: buccal, epicutaneous, epidural, infusion, inhalation, intraarterial, intracardial, intracerebroventricular, intradermal, intramuscular, intranasal, intraocular, intraperitoneal, intraspinal, intrathecal, intravenous, oral, parenteral, pulmonary, rectally via an enema or suppository, subcutaneous, subdermal, sublingual, transdermal, and transmucosal. The preferred routes of administration are intravenous injection or infusion. The administration can be local, where the composition is administered directly, close to, in the locality, near, at, about, or in the vicinity of, the site(s) of disease, e.g., tumor, or systemic, wherein the composition is given to the patient and passes through the body widely, thereby reaching the site(s) of disease. Local administration (e.g., injection) may be accomplished by administration to the cell, tissue, organ, and/or organ system, which encompasses and/or is affected by the disease, and/or where the disease signs and/or symptoms are active or are likely to occur (e.g., tumor site). Administration can be topical with a local effect, composition is applied directly where its action is desired (e.g., tumor site).

For each of the recited embodiments, the compounds can be administered by a variety of dosage forms as known in the art. Any biologically-acceptable dosage form known to persons of ordinary skill in the art, and combinations thereof, are contemplated. Examples of such dosage forms include, without limitation, chewable tablets, quick dissolve tablets, effervescent tablets, reconstitutable powders, elixirs, liquids, solutions, suspensions, emulsions, tablets, multilayer tablets, bi-layer tablets, capsules, soft gelatin capsules, hard gelatin capsules, caplets, lozenges, chewable lozenges, beads, powders, gum, granules, particles, microparticles, dispersible granules, cachets, douches, suppositories, creams, topicals, inhalants, aerosol inhalants, patches, particle inhalants, implants, depot implants, ingestibles, injectables (including subcutaneous, intramuscular, intravenous, and intradermal), infusions, and combinations thereof.

Other compounds which can be included by admixture are, for example, medically inert ingredients (e.g., solid and liquid diluent), such as lactose, dextrosesaccharose, cellulose, starch or calcium phosphate for tablets or capsules, olive oil or ethyl oleate for soft capsules and water or vegetable oil for suspensions or emulsions; lubricating agents such as silica, talc, stearic acid, magnesium or calcium stearate and/or polyethylene glycols; gelling agents such as colloidal clays; thickening agents such as gum tragacanth or sodium alginate, binding agents such as starches, arabic gums, gelatin, methylcellulose, carboxymethylcellulose or polyvinylpyrrolidone; disintegrating agents such as starch, alginic acid, alginates or sodium starch glycolate; effervescing mixtures; dyestuff; sweeteners; wetting agents such as lecithin, polysorbates or laurylsulphates; and other therapeutically acceptable accessory ingredients, such as humectants, preservatives, buffers and antioxidants, which are known additives for such formulations.

Liquid dispersions for oral administration can be syrups, emulsions, solutions, or suspensions. The syrups can contain as a carrier, for example, saccharose or saccharose with glycerol and/or mannitol and/or sorbitol. The suspensions and the emulsions can contain a carrier, for example a natural gum, agar, sodium alginate, pectin, methylcellulose, carboxymethylcellulose, or polyvinyl alcohol.

In further embodiments, the present invention provides kits including one or more containers comprising pharmaceutical dosage units comprising an effective amount of one or more VISTA antagonists of the present invention. Kits may include instructions, directions, labels, marketing information, warnings, or information pamphlets.

The amount of VISTA antagonist in a therapeutic composition according to any embodiments of this invention may vary according to factors such as the disease state, age, gender, weight, patient history, risk factors, predisposition to disease, administration route, pre-existing treatment regime (*e.g*., possible interactions with other medications), and weight of the individual. Dosage regimens may be adjusted to provide the optimum therapeutic response. For example, a single bolus may be administered, several divided doses may be administered over time, or the dose may be proportionally reduced or increased as indicated by the exigencies of therapeutic situation.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of antibodies, and fragments thereof, calculated to produce the desired therapeutic effect in association with the required pharmaceutical carrier. The specification for the dosage unit forms of the invention are dictated by and directly dependent on the unique characteristics of the antibodies, and fragments thereof, and the particular therapeutic effect to be achieved, and the limitations inherent in the art of compounding such an antibodies, and fragments thereof, for the treatment of sensitivity in individuals. In therapeutic use for treatment of conditions in mammals (e.g., humans) for which the antibodies and fragments thereof of the present invention or an appropriate pharmaceutical composition thereof are effective, the antibodies and fragments thereof of the present invention may be administered in an effective amount. The dosages as suitable for this invention may be a composition, a pharmaceutical composition or any other compositions described herein.

The dosage may be administered as a single dose, a double dose, a triple dose, a quadruple dose, and/or a quintuple dose. The dosages may be administered singularly, simultaneously, and sequentially.

The dosage form may be any form of release known to persons of ordinary skill in the art. The compositions of the present invention may be formulated to provide immediate release of the active ingredient or sustained or controlled release of the active ingredient. In a sustained release or controlled release preparation, release of the active ingredient may occur at a rate such that blood levels are maintained within a therapeutic range but below toxic levels over an extended period of time (*e.g*., 4 to 24 hours). The preferred dosage forms include immediate release, extended release, pulse release, variable release, controlled release, timed release, sustained release, delayed release, long acting, and combinations thereof, and are known in the art.

As defined herein, a therapeutically effective amount of VISTA antagonist peptide, analog, derivative or mimetic thereof (*i.e*., an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight.

The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of a peptide can include a single treatment or, preferably, can include a series of treatments.

In a preferred example, a subject is treated with peptide, analog, derivative or mimetic thereof in the range of between about 0.1 to 20 mg/kg body weight, one time per week for between about 1 to 10 weeks, preferably between 2 to 8 weeks, more preferably between about 3 to 7 weeks, and even more preferably for about 4, 5, or 6 weeks. It will also be appreciated that the effective dosage of antibody, protein, or polypeptide used for treatment may increase or decrease over the course of a particular treatment. Changes in dosage may result and become apparent from the results of diagnostic assays as described herein.

It will be appreciated that the pharmacological activity of the compositions may be monitored using standard pharmacological models that are known in the art. Furthermore, it will be appreciated that the compositions comprising a VISTA antagonist may be incorporated or encapsulated in a suitable polymer matrix or membrane for site-specific delivery, or may be functionalized with specific targeting agents capable of effecting site specific delivery. These techniques, as well as other drug delivery techniques are well known in the art. Determination of optimal dosages for a particular situation is within the capabilities of those skilled in the art. *See, e.g*., Grennaro (2005) [Ed.] Remington: The Science and Practice of Pharmacy [21st Ed.]

A peptide or derivative of this invention can be co-formulated with and/or coadministered with one or more additional therapeutic agents (*e.g*., an anti-cancer agent, an anti-viral agent, a cytokine and/or an immune agonist). Such combination therapies may require lower dosages of the peptide or derivative and/or the coadministered agents, thus avoiding possible toxicities or complications associated with the various monotherapies. There are a number of agents that may be advantageously combined with peptide or derivative of the invention and the selection of such agents will depend on the intended disease or condition to be treated. For example, the present invention includes combination therapies composed of a peptide or multimer, conjugate, or derivative of the invention that is capable of inducing or promoting a response against a cancerous or pre-cancerous condition and at least one anti-cancer agent. Accordingly, in particular embodiments, the instant peptide or derivative is used as an adjuvant therapy in the treatment of cancer. As another example, the invention embraces combination therapies that include a peptide or derivative of the invention that is capable of inducing or promoting a therapeutic response against a viral infection and at least one anti-viral agent. Exemplary therapeutic agents that may be contained in the compositions comprising the VISTA antagonist peptide, multimer, conjugate, or derivative include, e.g., CTLA-4-Ig, anti-PD-1, PD-L1 or PD-L2 fusion proteins and EGFR antagonists.

Anti-cancer agents include, but are not limited to, cytotoxic agents such as Vinca alkaloid, taxanes, and topoisomerase inhibitors; antisense nucleic acids such as augmerosen/G3139, LY900003 (ISIS 3521), ISIS 2503, OGX-011 (ISIS 112989), LE-AON/LEraf-AON (liposome encapsulated c-raf antisense oligonucleotide/ISIS-5132), MG98, and other antisense nucleic acids that target PKCα, clusterin, IGFBPs, protein kinase A, cyclin D1, or Bcl-2; anticancer nucleozymes such as angiozyme (Ribozyme Pharmaceuticals); tumor suppressor-encoding nucleic acids such as a p53, BRCA1, RB1, BRCA2, DPC4 (Smad4), MSH2, MLH1, and DCC; oncolytic viruses such as oncolytic adenoviruses and herpes viruses; anti-cancer immunogens such as a cancer antigen/tumor-associated antigen, e.g., an epithelial cell adhesion molecule (Ep-CAM/TACSTD1), mucin 1 (MUC1), carcinoembryonic antigen (CEA), tumor-associated glycoprotein 72 (TAG-72), gp100, Melan-A, MART-1, KDR, RCAS1, MDA7, cancer-associated viral vaccines, tumor-derived heat shock proteins, and the like; anti-cancer cytokines, chemokines, or combination thereof; inhibitors of angiogenesis, neovascularization, and/or other vascularization; and/or any other conventional anticancer agent including fluoropyrimidiner carbamates, non-polyglutamatable thymidylate synthase inhibitors, nucleoside analogs, antifolates, topoisomerase inhibitors, polyamine analogs, mTOR inhibitors, alkylating agents, lectin inhibitors, vitamin D analogs, carbohydrate processing inhibitors, anti-metabolism folate antagonists, thumidylate synthase inhibitors, antimetabolites, ribonuclease reductase inhibitors, dioxolate nucleoside analogs, and chemically modified tetracyclines.

Anti-viral agents of use in the invention include, but are not limited to, protease inhibitors (*e.g.,* acyclovir) in the context of HIV treatment or an anti-viral antibody (e.g., an anti-gp41 antibody in the context of HIV treatment; an anti-CD4 antibody in the context of the treatment of CMV, etc.). Numerous other types of anti-viral agents are known in the art.

Toxicity and therapeutic efficacy of the peptide or analog, derivative or mimetic can be determined by standard pharmaceutical procedures in cell cultures or experimental animals. The data obtained from the cell culture assays and animal studies can be used in formulating a range of dosage for use in humans. The dosage of such agents lies preferably within a range of circulating concentrations that include the ED₅₀ with little or no toxicity. The dosage may vary within this range depending upon the dosage form employed and the route of administration utilized. For any agent used in the methods of the invention, the therapeutically effective dose can be estimated initially from cell culture assays. A dose may be formulated in animal models to achieve a circulating plasma concentration range that includes the IC₅₀ (*i.e*., the concentration of the test compound which achieves a half-maximal inhibition of symptoms) as determined in cell culture. Such information can be used to more accurately determine useful doses in humans. Levels in plasma may be measured, for example, by high performance liquid chromatography.

### Use of VISTA Antagonists and Compositions comprising the same

The peptide or derivative of this invention finds use in inhibiting the activity of VISTA (*i.e*., PD-L3) thereby upregulating immune responses. Upregulation of immune responses can be in the form of enhancing an existing immune response or eliciting an initial immune response. For example, enhancing an immune response through inhibition of VISTA activity is useful in the prevention and/or treatment of infections with microbes, *e.g*., bacteria, viruses, or parasites, or in cases of immunosuppression and cancer.

Accordingly, the present invention includes the VISTA antagonist, or a composition comprising the same, for use in prophylactic and therapeutic methods for the prevention and treatment of cancer and infectious disease. Terms such as "treat," "treating" and "treatment" herein refer to the delivery of an effective amount of a peptide or derivative of this invention with the purpose of easing, ameliorating, or eradicating (curing) such symptoms or disease states already developed. The terms "prevent," "preventing" and "prevention" refer to the delivery of an effective amount of a peptide or derivative of this invention with the purpose of preventing any symptoms or disease state to develop. Thus, these terms are meant to include prophylactic treatment.

According to one embodiment, the invention provides an effective amount of an isolated VISTA antagonist disclosed herein or a composition containing said isolated VISTA antagonist for use in a method of treating or preventing cancer, inhibiting tumor invasion and/or cancer metastasis in a subject in need thereof, such as a mammalian subject, preferably a human subject. Optionally the subject has one or more precancerous lesions or is predisposed to cancer, e.g., as a result of genetic mutation, family history or exposure to a carcinogenic agent. In another embodiment the invention provides an effective amount of an isolated VISTA antagonist disclosed herein or a composition containing said isolated VISTA antagonist for use in a method of treating cancer in a subject, such as a mammalian subject, preferably a human subject, such as a human subject, who optionally has a detectable level of cancer cells. In accordance with these embodiments, the subject is to be administered a peptide or derivative of this invention in an amount sufficient to detectably reduce the development or progression of the cancer in the subject.

Cancers are generally composed of single or several clones of cells that are capable of partially independent growth in a host (*e.g*., a benign tumor) or fully independent growth in a host (malignant cancer). Cancer cells are cells that divide and reproduce abnormally with uncontrolled growth.

Cancer cells arise from host cells via neoplastic transformation (*i.e*., carcinogenesis). Terms such as "preneoplastic," "premalignant" and "precancerous" with respect to the description of cells and/or tissues herein refer to cells or tissues having a genetic and/or phenotypic profile that signifies a significant potential of becoming cancerous. Usually such cells can be characterized by one or more differences from their nearest counterparts that signal the onset of cancer progression or significant risk for the start of cancer progression. Such precancerous changes, if detectable, can usually be treated with excellent results.

In general, a precancerous state will be associated with the incidence of neoplasm(s) or preneoplastic lesion(s). Examples of known and likely preneoplastic tissues include ductal carcinoma in situ (DCIS) growths in breast cancer, cervical intra-epithelial neoplasia (CIN) in cervical cancer, adenomatous polyps of colon in colorectal cancers, atypical adenomatous hyperplasia in lung cancers, and actinic keratosis (AK) in skin cancers. Pre-neoplastic phenotypes and genotypes for various cancers, and methods for assessing the existence of a preneoplastic state in cells, have been characterized. See, e.g., Medina (2000) J. Mammary Gland Biol. Neoplasia 5(4): 393-407; Krishnamurthy, et al. (2002) Adv. Anat. Pathol. 9(3):185-97; Ponten (2001) Eur. J. Cancer Suppl 8:S97-113; Niklinski, et al. (2001) Eur. J. Cancer Prev. 10(3):213-26; Walch, et al. Pathobiology (2000) 68(1):9-17; Busch (1998) Cancer Surv. 32:149-79.

Gene expression profiles can increasingly be used to differentiate between normal, precancerous, and cancer cells. For example, familial adenomatous polyposis genes prompt close surveillance for colon cancer; mutated p53 tumor-suppressor gene flags cells that are likely to develop into aggressive cancers; osteopontin expression levels are elevated in premalignant cells, and increased telomerase activity also can be a marker of a precancerous condition (*e.g*., in cancers of the bladder and lung). In one aspect, the invention relates to the treatment of precancerous cells. In another aspect, the invention relates to the preparation of medicaments for treatment of precancerous cells.

In general, a peptide or derivative of this invention can be for use in treating subjects suffering from any stage of cancer (and to prepare medicaments for reduction, delay, or other treatment of cancer). Effective treatment of cancer (and thus the reduction thereof) can be detected by any variety of suitable methods. Methods for detecting cancers and effective cancer treatment include clinical examination (symptoms can include swelling, palpable lumps, enlarged lymph nodes, bleeding, visible skin lesions, and weight loss); imaging (X-ray techniques, mammography, colonoscopy, computed tomography (CT and/or CAT) scanning, magnetic resonance imaging (MRI), etc.); immunodiagnostic assays (*e.g*., detection of CEA, AFP, CA125, etc.); antibody-mediated radioimaging; and analyzing cellular/tissue immunohistochemistry. Other examples of suitable techniques for assessing a cancerous state and effective cancer treatment include PCR and RT-PCR (*e.g*., of cancer cell associated genes or "markers"), biopsy, electron microscopy, positron emission tomography (PET), computed tomography, magnetic resonance imaging (MRI), karyotyping and other chromosomal analysis, immunoassay/immunocytochemical detection techniques (*e.g.,* differential antibody recognition), histological and/or histopathologic assays (*e.g*., of cell membrane changes), cell kinetic studies and cell cycle analysis, ultrasound or other sonographic detection techniques, radiological detection techniques, flow cytometry, endoscopic visualization techniques, and physical examination techniques.

In general, delivering a peptide or derivative of this invention to a subject (either by direct administration or expression from a nucleic acid) can be used to reduce, treat, prevent, or otherwise ameliorate any aspect of cancer in a subject. In this respect, treatment of cancer can include, *e.g*., any detectable decrease in the rate of normal cells transforming to neoplastic cells (or any aspect thereof), the rate of proliferation of pre-neoplastic or neoplastic cells, the number of cells exhibiting a pre-neoplastic and/or neoplastic phenotype, the physical area of a cell media (*e.g*., a cell culture, tissue, or organ) containing pre-neoplastic and/or neoplastic cells, the probability that normal cells and/or preneoplastic cells will transform to neoplastic cells, the probability that cancer cells will progress to the next aspect of cancer progression (*e.g*., a reduction in metastatic potential), or any combination thereof. Such changes can be detected using any of the above-described techniques or suitable counterparts thereof known in the art, which typically are applied at a suitable time prior to the administration of a therapeutic regimen so as to assess its effectiveness. Times and conditions for assaying whether a reduction in cancer has occurred will depend on several factors including the type of cancer, type and amount of peptide, related composition, or combination composition being delivered to the host.

The VISTA antagonist or compositions of the invention can be for use in treating a variety of cancers. Forms of cancer that may be treated by the delivery or administration of a peptide or derivative of this invention and combination therapies containing the same include squamous cell carcinoma, leukemia, acute lymphocytic leukemia, acute lymphoblastic leukemia, B-cell lymphoma, T-cell lymphoma, Hodgkins lymphoma, non-Hodgkins lymphoma, hairy cell lymphoma, Burketts lymphoma, acute or chronic myelogenous leukemias, promyelocytic leukemia, fibrosarcoma, rhabdomyoscarcoma, melanoma, seminoma, teratocarcinoma, neuroblastoma, glioma, astrocytoma, neuroblastoma, glioma, schwannomas; fibrosarcoma, rhabdomyoscaroma, osteosarcoma, melanoma, xeroderma pigmentosum, keratoacanthoma, seminoma, thyroid follicular cancer, and teratocarcinoma. The compositions of this invention also can be useful in the treatment of other carcinomas of the bladder, breast, colon, kidney, liver, lung, ovary, prostate, pancreas, stomach, cervix, thyroid or skin. Compositions of this invention also may be useful in treatment of other hematopoietic tumors of lymphoid lineage, other hematopoietic tumors of myeloid lineage, other tumors of mesenchymal origin, other tumors of the central or peripheral nervous system, and/or other tumors of mesenchymal origin. Advantageously, the VISTA antagonists or compositions for use according to the invention also may be useful in reducing cancer progression in prostate cancer cells, melanoma cells (e.g., cutaneous melanoma cells, ocular melanoma cells, and/or lymph node-associated melanoma cells), breast cancer cells, colon cancer cells, and lung cancer cells. The VISTA antagonists or compositions for use according to the invention can be used to treat both tumorigenic and non-tumorigenic cancers (e.g., non-tumor-forming hematopoietic cancers). The VISTA antagonists or compositions for use according to the invention are particularly useful in the treatment of epithelial cancers (e.g., carcinomas) and/or colorectal cancers, breast cancers, lung cancers, vaginal cancers, cervical cancers, and/or squamous cell carcinomas (e.g., of the head and neck).
Additional potential targets include sarcomas and lymphomas. Additional advantageous targets include solid tumors and/or disseminated tumors (*e.g*., myeloid and lymphoid tumors, which can be acute or chronic).

The present invention also provides an effective amount of an isolated VISTA antagonist or a composition containing said isolated VISTA antagonist for use in enhancing anti-cancer or anti-tumor immunity in a subject in need thereof.

In addition to cancer treatment, the present invention also features VISTA antagonists or compositions for use in treating a pathogen infection, i.e., a bacterial, viral, parasitic or fungal infection, in a subject or host. This use involves administering or otherwise delivering an effective amount of a peptide or derivative of this invention so as to reduce the severity, spread, symptoms, or duration of such infection. Such pathogen infections include, but are not limited to diseases caused by bacteria, protozoa, fungi, parasites, or viruses.

In particular embodiments, a viral infection is treated. Any virus normally associated with the activity of effector lymphocytes can be treated by the method. For example, the VISTA antagonists or compositions can be for use to treat infection by one or more viruses selected from hepatitis type A, hepatitis type B, hepatitis type C, influenza, varicella, adenovirus, herpes simplex type I (HSV-1), herpes simplex type 2 (HSV-2), rinderpest, rhinovirus, echovirus, rotavirus, respiratory syncytial virus, papilloma virus, papilloma virus, cytomegalovirus (CMV, *e.g*., HCMV), echinovirus, arbovirus, huntavirus, coxsackie virus, mumps virus, measles virus, rubella virus, polio virus, and/or human immunodeficiency virus type I or type 2 (HIV-1, HIV-2). This practice may result in a reduction in the titer of virus (viral load), reduction of the number of virally infected cells, etc.

In addition to pathogen infections, a peptide or derivative of this invention can be administered or otherwise delivered to a subject in association with the treatment of immunoproliferative diseases, immunodeficiency diseases, autoimmune diseases, inflammatory responses, and/or allergic responses.
Moreover, the invention also provides an effective amount of an isolated VISTA antagonist or a composition containing said isolated VISTA antagonist for use in blocking, inhibiting or neutralizing VISTA-mediated T cell suppression and/or stimulating an immune response in a subject in need thereof. This may be useful for treating a subject with a one or more of a bacterial, viral, parasitic and fungal infections and/or cancer.

### Examples

The invention now being generally described, it will be more readily understood by reference to the following examples, which are included merely for purposes of illustration of certain aspects and embodiments of the present invention, and are not intended to limit the invention. The invention is defined by the claims.

### Example 1: Materials and Methods

*Peptide Synthesis.* AP1049 (SSACDWIKRSCH-amide, wherein Cys4-Cys11 form a disulfide bridge; SEQ ID NO:1 (Ser-Ser-Ala-Cys-Asp-Trp-Ile-Lys-Arg-Ser-Cys-His)) and scrambled negative control sequence (SSACKSWRDICH-amide, wherein Cys4-Cys11 form a disulfide bridge; SEQ ID NO:2) were prepared using standard Fmoc-based solid-phase peptide synthesis (SPPS). The peptides were purified via HPLC, and analyzed by mass spectrometric using the liquid chromatography-mass spectrometry (LC-MS) and matrix-assisted laser desorption/ionization (MALDI)

*Peptide Discovery Using Phage Display.* An M13 phage peptide library was provided by Dr. Brian Kay (U. Illinois-Chicago). The VISTA protein required for both the phage display biopanning experiments and the confirmatory ELISA binding experiments was prepared by conventional recombinant protein techniques.

*T Cell Proliferation Assay.* An VISTA-Ig fusion protein or control Ig fusion protein was co-absorbed to a cell-culture plate together with the polyclonal T cell receptor (TCR) stimuli (i.e., anti-CD3 antibody). To evaluate the activity of VISTA-specific peptides, peptides (VISTA specific and scrambled control) were added as a soluble reagent to the culture on day 0, and T cell proliferation and cytokine production were analyzed after 3-4 days.

*T Cell Priming Assay.* VISTA is known to suppress T cell priming when expressed on antigen-presenting cells (APCs). VISTA-expressing myeloid APCs (Cd11b^{hi} MHCII⁺ myeloid cells) were purified from mice spleen, FACS sorted, and irradiated (2500 rads). To test the activity of VISTA-specific peptides, transgenic T cells such as OT-II were stimulated ex *vivo* with VISTA-expressing APCs and cognate antigen chicken ovalbumin (15 ng/mL). VISTA-specific peptide or control scramble peptide was added to the cell culture. T cell proliferation and cytokine production was evaluated after 3-5 days of culture. As an additional specificity control, VISTA-negative parent cell line A20 or APCs purified from VISTA knockout mice were used.

*Foxp3+CD4+ Regulatory T Cell (Treg) Suppression Assay.* VISTA plays a role in the suppressive function of Foxp3+ CD4+ regulatory T cells (Tregs), as VISTA-blocking monoclonal antibody partially reverses Treg suppressive activity in the *in vitro* Treg suppression assay. This assay includes antigen presenting cells, purified Foxp3+ CD4+ Tregs, and Foxp3- CD4+ naive T cells, which are stimulated by the polyclonal TCR stimuli. To examine the activity of VISTA-specific peptides, peptides (VISTA specific and scrambled control) were added to the Treg suppression assay on day 0. T cell proliferation and cytokine production were measured on day +3.

*Model of Experimental Autoimmune Encephalomyelitis (EAE), a Murine Autoimmune Inflammatory Disease Model for Human Multiple Sclerosis.* It has been shown that VISTA-blocking monoclonal antibody significantly accelerates disease onset, as well as exacerbates disease severity in a passive transfer EAE model. In this model, MOG-specific encephalitogenic CD4+ T cells are first primed in donor mice upon immunization with MOG peptide, and then purified and ex *vivo* expanded in the presence of MOG peptide and cytokines (IL23, TGFβ, IL6 and IL1b). Expanded encephalogenic CD4+ T cells are transferred into naive recipients to induce disease. To evaluate the activity of VISTA-specific peptides, peptides (VISTA specific and scrambled control) are administered via intraperitoneal injections to mice either prophylactically (starting from day 2) or therapeutically (starting from day +7 during disease onset), and continuously every 2 days for the entire duration of the experiment. Disease severity is evaluated according to the established protocol.

*Murine Tumor Models.* It has been demonstrated that VISTA suppresses tumor-specific T cell responses. VISTA blockade via VISTA-specific monoclonal antibody significantly enhances anti-tumor immune responses and inhibits tumor progression in murine tumor models such as the B16 melanoma model. The activity of VISTA-specific peptides can be evaluated *in vivo* using this tumor model. Mice are inoculated on the flank with B16 tumor cells (15,000 cells) on day 0. Peptides (VISTA specific and scrambled control) are administered via intraperitoneal injections to mice either prophylactically (starting from day 2) or therapeutically (i.e., when tumors are palpable), and continuously every 2 days for the entire duration of the experiment. Tumor growth is measured every 2-3 days with a caliper.

### Example 2: Enhancement of T Cell Proliferation

VISTA⁺CD11b⁺ monocytes were enriched from naive splenocytes using CD11b magnetic beads (Miltenyi). VISTA⁺CD11b^{hi} MHCII⁺ myeloid APCs were FACS sorted, irradiated (2500 rads), and used as antigen-presenting cells to stimulate OT-II transgenic CD4⁺ T cells in the presence of OVA peptide. Control-Ig, monoclonal antibody specific for VISTA and PD-L1 (30 µg/mL), or VISTA-specific peptide (100 µg/mL) were added as indicated. Cell proliferation was measured by tritium incorporation during the last 8 hours of a 72-hour assay. This analysis indicated that T cell proliferation was enhanced in the presence of VISTA or PD-L1 neutralizing monoclonal antibodies, or the AP1049 peptide (Figure 1). In fact, the AP1049 peptide stimulated T cell proliferation much better than either of the monoclonal antibodies, indicating that the peptide possesses strong antagonistic activity against VISTA.

### Example 3: Enhancement of anti-tumor immunity

Immunogenic bladder carcinoma tumors (MB49) were inoculated in female mice. AP1049 was tested for its ability to slow tumor growth and/or facilitate tumor regression. The readout for this assay was tumor growth.

MB49 tumors were inoculated in female mice (300k) via intradermal (i.d.) inoculation, which facilitates measurement of tumor size. Mice were treated with either PBS (control) or VISTA antagonist peptide (AP1049), via daily injections around tumor mass starting on day+1 and continuing for 2 weeks. Tumor size was measured by caliper every 2-3 days.

Using these methods slowed tumor growth and/or tumor regression in mice treated with AP1049 was obtained as compared with mice treated with control.

As shown in Figure 2, AP1049 treatment reduced tumor growth in the MB49 tumor model, indicating that the peptide may bind to the critical/active site of VISTA and block the immune-suppressive function of VISTA.

## Claims

1. An isolated VISTA antagonist selected from the following:
(i) one comprising a peptide which is identical to the amino acid sequence of SEQ ID NO:1 (Ser-Ser-Ala-Cys-Asp-Trp-Ile-Lys-Arg-Ser-Cys-His), or a multimer, conjugate, or derivative thereof, wherein the derivative thereof contains a modification of one or more amino acid residues or a linker group or other covalently linked group;
(ii) one comprising a peptide which is identical to the amino acid sequence of SEQ ID NO:1 (Ser-Ser-Ala-Cys-Asp-Trp-Ile-Lys-Arg-Ser-Cys-His), or which comprises a peptide having an amino acid sequence that differs from SEQ ID NO: 1 by at most 2 amino acid residues, or a multimer, conjugate, or derivative thereof, wherein the derivative thereof contains a modification of one or more amino acid residues or a linker group or other covalently linked group;
(iii) one which comprises a peptide having an amino acid sequence that differs from SEQ ID NO:1 by at most 1 amino acid residue, or a multimer, conjugate, or derivative thereof, wherein the derivative thereof contains a modification of one or more amino acid residues or a linker group or other covalently linked group;
(iv) one comprising a peptide which is identical to the amino acid sequence of SEQ ID NO:1 (Ser-Ser-Ala-Cys-Asp-Trp-Ile-Lys-Arg-Ser-Cys-His) or a multimer, or conjugate thereof;
(v) one which consists of a peptide which is identical to the amino acid sequence of SEQ ID NO:1 (Ser-Ser-Ala-Cys-Asp-Trp-Ile-Lys-Arg-Ser-Cys-His);
(vi) one according to any one of (i) to (v) wherein the cysteine residues at positions 4 and 11 of SEQ ID NO:1 (Ser-Ser-Ala-Cys-Asp-Trp-Ile-Lys-Arg-Ser-Cys-His), or their corresponding position in a variant of said peptide, form a disulfide bridge;
(vii) one according to any one of (i) to (vi) which has been modified to improve binding affinity and/or stability;
(viii) one according to (vii) wherein the modification is selected from the group consisting of PEG, acetylation, XTEN, albumin and multimerization;
(ix) one according to any one of (i) to (viii) which is directly or indirectly attached to an immunoglobulin polypeptide or a fragment thereof;
(x) one according to (ix) wherein said immunoglobulin polypeptide or fragment thereof comprises a human IgG1, IgG2, IgG3 or IgG4 constant region or fragment thereof;
(xi) one according to (x) wherein said immunoglobulin polypeptide comprises a human IgG1, constant region or fragment thereof;
(xii) one according to any one of (i) to (xi) which comprises 2, 3, 4, 5, 6, 7 or more copies of said peptide;
(xiii) one according to any one of (i) to (xi) which comprises another moiety that targets said peptide to a target site;
(xiv) one according to (xiii) wherein the targeting moiety is selected from an antibody or ligand that binds to an antigen, a receptor expressed by the target cell or an infectious agent;
(xv) one according to any one of (i) to (xiv) wherein the VISTA antagonist is attached to another moiety or another copy of said VISTA antagonist via a linker;
(xvi) one according to (xv) wherein the linker is a peptide that permits the VISTA antagonist to interact with VISTA expressed on the surface of a target cell;
(xvii) one according to any one of (i) to (xvi) which is directly or indirectly attached to a detectable label or therapeutic agent;
(xviii) one according to any one of (i) to (xvii) which binds to the extracellular domain of VISTA and disrupts its interaction with a VISTA receptor;
(xix) one according to any one of (i) to (xviii) which reduces or inhibits VISTA-mediated T cell suppression; and/or
(xx) one according to any one of (i) to (xix) which elicits anti-tumor and/or anti-viral activity.

2. A composition suitable for therapeutic, prophylactic or diagnostic use comprising a therapeutically, prophylactically or diagnostically effective amount of an isolated VISTA antagonist according to claim 1.

3. The composition of claim 2, further comprising a pharmaceutically acceptable carrier, diluent, solubilizer, preservative or mixture thereof.

4. The composition of claim 2 or 3, further comprising another therapeutic agent.

5. The composition of claim 4 wherein the other therapeutic agent is an anti-cancer agent, an anti-viral agent, a cytokine or an immune agonist optionally selected from CTLA-4-Ig, anti-PD-1, PD-L1 or PD-L2 fusion proteins, and EGFR antagonists.

6. A composition according to any one of claims 2 to 5, which is suitable for subcutaneous administration or intravenous administration.

7. An isolated nucleic acid sequence encoding a VISTA antagonist according to claim 1 or a vector or host cell containing said nucleic acid sequence, wherein said host cell is optionally a mammalian cell, bacterial cell, a fungal cell, a yeast cell, an avian cell or an insect cell.

8. A method of expressing a VISTA antagonist comprising culturing a host cell according to claim 7 under conditions that provide for expression of said VISTA antagonist.

9. A composition comprising an effective amount of a VISTA antagonist according to claim 1 for:
(i) use in blocking, inhibiting or neutralizing VISTA-mediated T cell suppression;
(ii) use in stimulating an immune response in a subject in need thereof, optionally one with an infection selected from the group consisting of bacterial, viral, parasitic and fungal infections;
(iii) use in treating cancer in a subject in need thereof;
(iv) use in enhancing anti-cancer or anti-tumor immunity in a subject in need thereof;
(v) use in treating or preventing cancer, inhibiting tumor invasion and/or cancer metastasis in a subject in need thereof;
(vi) use of (iii), (iv) or (v) wherein the cancer is selected from the group consisting of carcinoma, lymphoma, blastoma, sarcoma, leukemia, lymphoid malignancies, melanoma, squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma, head and neck cancer, B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenström's Macroglobulinemia); chronic lymphocytic leukemia (CLL); acute lymphoblastic leukemia (ALL); Hairy cell leukemia; chronic myeloblastic leukemia; post-transplant lymphoproliferative disorder (PTLD), abnormal vascular proliferation associated with phakomatoses, edema (such as that associated with brain tumors), and Meigs' syndrome; or
(vii) use in treating or preventing a viral infection in a subject in need thereof.

10. The composition for use of claim 9, wherein the subject has
(i) a bacterial infection caused by at least one bacterium selected from the group consisting of *Bordetella*, *Borrelia*, *Brucella*, *Burkholderia*, *Campylobacter*, *Chlamydia*, *Clostridium*, *Corynebacterium*, *Enterobacter*, *Enterococcus*, *Erwinia*, *Escherichia*, *Francisella*, *Haemophilus*, *Helicobacter*, *Legionella*, *Leptospira*, *Listeria*, *Mycobacterium*, *Mycoplasma*, *Neisseria*, *Pasteurella*, *Pelobacter*, *Pseudomonas*, *Rickettsia*, *Salmonella*, *Serratia*, *Shigella*, *Staphylococcus, Streptococcus, Treponema, Vibrio, Yersinia* and *Xanthomonas*;
(ii) a viral infection caused by at least one virus selected from the group consisting of *Adenoviridae*, *Papillomaviridae*, *Polyomaviridae*, *Herpesviridae*, *Poxviridae*, *Hepadnaviridae*, *Parvoviridae*, *Astroviridae*, *Caliciviridae*, *Picornaviridae*, *Coronaviridae*, *Flaviviridae*, *Retroviridae*, *Togaviridae*, *Arenaviridae*, *Bunyaviridae*, *Filoviridae*, *Orthomyxoviridae*, *Paramyxoviridae*, *Rhabdoviridae*, and Reoviridae;
(iii) a viral infection caused by an adenovirus, herpes simplex type I, herpes simplex type 2, Varicella-zoster virus, Epstein-Barr virus, cytomegalovirus, herpesvirus type 8, papillomavirus, BK virus, JC virus, smallpox, Hepatitis B, bocavirus, parvovirus B19, astrovirus, Norwalk virus, coxsackievirus, Hepatitis A, poliovirus, rhinovirus, severe acute respiratory syndrome virus, Hepatitis C, yellow fever, dengue virus, West Nile virus, rubella, Hepatitis E, human immunodeficiency virus (HIV), influenza, Guanarito virus, Junin virus, Lassa virus, Machupo virus, Sabia virus, Crimean-Congo hemorrhagic fever virus, Ebola virus, Marburg virus, measles virus, mumps virus, parainfluenza, respiratory syncytial virus, human metapneumovirus, Hendra virus, Nipah virus, rabies, Hepatitis D, rotavirus, orbivirus, coltivirus or Banna virus;
(iv) a fungal infection selected from the group consisting of thrush, candidiasis, cryptococcosis, histoplasmosis, blastomycosis, aspergillosis, coccidioidomycosis, paracoccidiomycosis, sporotrichosis, zygomycosis, chromoblastomycosis , lobomycosis, mycetoma, onychomycosis, piedra pityriasis versicolor, tinea barbae, tinea capitis, tinea corporis, tinea cruris, tinea favosa, tinea nigra, tinea pedis, otomycosis, phaeohyphomycosis, or rhinosporidiosis; or
(v) a parasitic infection caused by at least one parasite selected from the group consisting *of Entamoeba histolytica*, *Giardia lamblia*, *Cryptosporidium muris*, *Trypanosomatidae gambiense*, *Trypanosomatidae rhodesiense*, *Trypanosomatidae cruzi*, *Leishmania mexicana*, *Leishmania braziliensis*, *Leishmania tropica*, *Leishmania donovani*, *Toxoplasma gondii*, *Plasmodium vivax*, *Plasmodium ovale, Plasmodium malariae*, *Plasmodium falciparum*, *Trichomonas vaginalis*, *Histomonas meleagridis*; *Secementea*; *Trichuris trichiura*, *Ascaris lumbricoides*, *Enterobius vermicularis*, *Ancylostoma duodenale*, *Necator americanus*, *Strongyloides stercoralis*, *Wuchereria bancrofti*, *Dracunculus medinensis*; blood flukes, liver flukes, intestinal flukes, lung flukes; *Schistosoma mansoni*, *Schistosoma haematobium*, *Schistosoma japonicum*, Fasciola hepatica, *Fasciola gigantica*, *Heterophyes heterophyes*, and *Paragonimus westermani.*

11. A VISTA antagonist according to claim 1 for:
(i) use in blocking, inhibiting or neutralizing VISTA-mediated T cell suppression,
(ii) use in stimulating an immune response in a subject in need thereof, optionally one with an infection selected from the group consisting of bacterial, viral, parasitic and fungal infections;
(iii) use in treating cancer in a subject in need thereof;
(iv) use in enhancing anti-cancer or anti-tumor immunity in a subject in need thereof,
(v) use in treating or preventing cancer, inhibiting tumor invasion and/or cancer metastasis in a subject in need thereof;
(vi) the use of (iii), (iv) or (v) wherein the cancer is selected from the group consisting of carcinoma, lymphoma, blastoma, sarcoma, leukemia, lymphoid malignancies, melanoma, squamous cell cancer, lung cancer (including small-cell lung cancer, non-small cell lung cancer, adenocarcinoma of the lung, and squamous carcinoma of the lung) cancer of the peritoneum, hepatocellular cancer, gastric or stomach cancer (including gastrointestinal cancer) pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma, head and neck cancer, B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenström's Macroglobulinemia) chronic lymphocytic leukemia (CLL); acute lymphoblastic leukemia (ALL); Hairy cell leukemia; chronic myeloblastic leukemia; post-transplant lymphoproliferative disorder (PTLD), abnormal vascular proliferation associated with phakomatoses, edema (such as that associated with brain tumors), and Meigs' syndrome; or
(vii) use in treating or preventing a viral infection in a subject in need thereof.

12. The VISTA antagonist for use according to claim 11, further comprising the use of another therapeutic agent, wherein said VISTA antagonist peptide and other therapeutic may be separately or jointly administered, at the same or different times.

13. The VISTA antagonist for use of claim 12, wherein the other therapeutic agent is an anti-cancer agent, an anti-viral or other anti-infectious agent, a cytokine or an immune agonist such as CTLA-4-Ig, anti-PD-1, PD-L1 or PD-L2 fusion proteins, or an EGFR antagonist.

14. Use of an isolated VISTA antagonist in a method for mapping the active site of VISTA, comprising: (a) incubating an isolated VISTA fusion protein with the isolated VISTA antagonist comprising a peptide that is identical to the amino acid sequence of SEQ ID NO:1 (Ser-Ser-Ala-Cys-Asp-Trp-Ile-Lys-Arg-Ser-Cys-His), or which comprises a peptide having an amino acid sequence which differs from SEQ ID NO:1 by at most 2 amino acid residues or an multimer, conjugate, or derivative thereof, wherein the derivative thereof contains a modification of one or more amino acid residues or a linker group or other covalently linked group; and (b) determining the binding site of the isolated VISTA antagonist.

15. The use of claim 14, wherein the active site of VISTA binds to a VISTA receptor and mediates immune suppression or step (b) comprises domain deletion, domain swapping, amino acid mutagenesis, foot printing, NMR, X-ray crystallography or homology modeling.

## Patentansprüche

1. Isolierter VISTA-Antagonist, ausgewählt aus den folgenden:
(i) einem, umfassend ein Peptid, das mit der Aminosäuresequenz von SEQ ID NO: 1 (Ser-Ser-Ala-Cys-Asp-Trp-Ile-Lys-Arg-Ser-Cys-His) identisch ist, oder ein Multimer, Konjugat, oder Derivat davon, wobei das Derivat davon eine Modifikation von einem oder mehreren Aminosäureresten oder eine Linkergruppe oder eine andere kovalent gebunden Gruppe enthält;
(ii) einem, umfassend ein Peptid, das mit der Aminosäuresequenz von SEQ ID NO: 1 (Ser-Ser-Ala-Cys-Asp-Trp-Ile-Lys-Arg-Ser-Cys-His) identisch ist, oder umfassend ein Peptid mit einer Aminosäuresequenz, die sich von SEQ ID NO: 1 um höchstens 2 Aminosäurereste unterscheidet, oder ein Multimer, Konjugat, oder Derivat davon, wobei das Derivat davon eine Modifikation von einem oder mehreren Aminosäureresten oder eine Linkergruppe oder eine andere kovalent gebunden Gruppe enthält;
(iii) einem, umfassend ein Peptid mit einer Aminosäuresequenz, die sich von SEQ ID NO: 1 um höchstens 1 Aminosäurerest unterscheidet, oder ein Multimer, Konjugat, oder Derivat davon, wobei das Derivat davon eine Modifikation von einem oder mehreren Aminosäureresten oder eine Linkergruppe oder eine andere kovalent gebunden Gruppe enthält;
(iv) einem, umfassend ein Peptid, das mit der Aminosäuresequenz von SEQ ID NO: 1 (Ser-Ser-Ala-Cys-Asp-Trp-Ile-Lys-Arg-Ser-Cys-His) identisch ist, oder ein Multimer oder Konjugat davon;
(v) einem, der aus einem Peptid besteht, das mit der Aminosäuresequenz von SEQ ID NO: 1 (Ser-Ser-Ala-Cys-Asp-Trp-Ile-Lys-Arg-Ser-Cys-His) identisch ist;
(vi) einem nach einem aus (i) bis (v), wobei die Cysteinreste an den Positionen 4 und 11 von SEQ ID NO: 1 (Ser-Ser-Ala-Cys-Asp-Trp-Ile-Lys-Arg-Ser-Cys-His) oder ihrer entsprechenden Position in einer Variante des Peptids eine Disulfidbrücke bilden;
(vii) einem nach einem aus (i) bis (vi), der modifiziert wurde, um die Bindungsaffinität und/oder Stabilität zu verbessern;
(viii) einem nach (vii), wobei die Modifikation aus der aus PEG, Acetylierung, XTEN, Albumin und Multimerisierung bestehenden Gruppe ausgewählt ist;
(ix) einem nach einem von (i) bis (viii), der direkt oder indirekt an ein Immunglobulinpolypeptid oder ein Fragment davon gebunden ist;
(x) einem nach (ix), wobei das Immunglobulinpolypeptid oder das Fragment davon eine konstante Region von menschlichem IgG1, IgG2, IgG3 oder IgG4 oder ein Fragment davon umfasst;
(xi) einem nach (x), wobei das Immunglobulinpolypeptid ein menschliches IgG1, eine konstante Region oder ein Fragment davon umfasst;
(xii) einem nach einem aus (i) bis (xi), der 2, 3, 4, 5, 6, 7 oder mehr Kopien des Peptids umfasst;
(xiii) einem nach einem aus (i) bis (xi), der eine weitere Einheit umfasst, die das Peptid an eine Zielstelle steuert;
(xiv) einem nach (xiii), wobei die Zielsteuerungseinheit aus einem Antikörper oder einem Liganden ausgewählt ist, der an ein Antigen, einen von der Zielzelle exprimierten Rezeptor oder einen Infektionserreger bindet;
(xv) einem nach einem aus (i) bis (xiv), wobei der VISTA-Antagonist an eine andere Einheit oder eine andere Kopie des VISTA-Antagonisten über einen Linker gebunden ist;
(xvi) einem nach (xv), wobei der Linker ein Peptid ist, das dem VISTA-Antagonisten die Interaktion mit auf der Oberfläche einer Zielzelle exprimiertem VISTA ermöglicht;
(xvii) einem nach einem aus (i) bis (xvi), der direkt oder indirekt an eine nachweisbare Markierung oder ein therapeutisches Mittel gebunden ist;
(xviii) einem nach einem aus (i) bis (xvii), der an die extrazelluläre Domäne von VISTA bindet und dessen Wechselwirkung mit einem VISTA-Rezeptor unterbricht;
(xix) einem nach einem aus (i) bis (xviii), der die VISTA-vermittelte T-Zell-Suppression verringert oder hemmt, und/oder
(xx) einem nach einem aus (i) bis (xix), der eine Anti-Tumor- und/oder Anti-Virus-Aktivität hervorruft.

2. Zusammensetzung, die zur therapeutischen, prophylaktischen oder diagnostischen Verwendung geeignet, umfassend eine therapeutisch, prophylaktisch oder diagnostisch wirksame Menge eines isolierten VISTA-Antagonisten nach Anspruch 1.

3. Zusammensetzung nach Anspruch 2, die ferner einen pharmazeutisch annehmbaren Träger, ein pharmazeutisch annehmbares Verdünnungsmittel, einen pharmazeutisch annehmbaren Lösungsvermittler, ein pharmazeutisch annehmbares Konservierungsmittel oder ein Gemisch davon umfasst.

4. Zusammensetzung nach Anspruch 2 oder 3, die ferner ein weiteres therapeutisches Mittel umfasst.

5. Zusammensetzung nach Anspruch 4, wobei das andere therapeutische Mittel ein Antikrebsmittel, ein antivirales Mittel, ein Zytokin oder ein Immunagonist, optional ausgewählt aus CTLA-4-Ig, Anti-PD-1-, PD-L1- oder PD-L2-Fusionsproteinen und EGFR-Antagonisten, ist.

6. Zusammensetzung nach einem der Ansprüche 2 bis 5, die zur subkutanen Verabreichung oder zur intravenösen Verabreichung geeignet ist.

7. Isolierte Nukleinsäuresequenz, die einen VISTA-Antagonisten nach Anspruch 1 codiert, oder Vektor oder Wirtszelle, der/die die Nukleinsäuresequenz enthält, wobei die Wirtszelle optional eine Säugerzelle, eine Bakterienzelle, eine Pilzzelle, eine Hefezelle, eine Vogelzelle oder eine Insektenzelle ist.

8. Verfahren zum Exprimieren eines VISTA-Antagonisten, umfassend das Kultivieren einer Wirtszelle nach Anspruch 7 unter Bedingungen, die die Expression des VISTA-Antagonisten bereitstellen.

9. Zusammensetzung, umfassend eine wirksame Menge eines VISTA-Antagonisten nach Anspruch 1, zur:
(i) Verwendung bei der Blockierung, Hemmung oder Neutralisierung von VISTA-vermittelter T-Zell-Suppression,
(ii) Verwendung bei der Stimulation einer Immunantwort in einem Individuum, das dessen bedarf, optional einem Individuum mit einer Infektion, ausgewählt aus der Gruppe, bestehend aus Bakterien-, Virus-, Parasiten- und Pilzinfektionen;
(iii) Verwendung bei der Behandlung von Krebs in einem Individuum, das dessen bedarf;
(iv) Verwendung bei der Verstärkung von Anti-Krebs- oder Anti-Tumor-Immunität in einem Individuum, das dessen bedarf;
(v) Verwendung bei der Behandlung oder Vorbeugung von Krebs, Hemmung der Tumorinvasion und/oder Krebsmetastasierung in einem Individuum, das dessen bedarf;
(vi) Verwendung nach (iii), (iv) oder (v), wobei der Krebs aus der Gruppe ausgewählt ist, bestehend aus Karzinom, Lymphom, Blastom, Sarkom, Leukämie, Lymphmalignomen, Melanom, Plattenepithelzellkrebs, Lungenkrebs (einschließlich kleinzelligem Lungenkrebs, nicht-kleinzelligem Lungenkrebs, Adenokarzinom der Lunge und Plattenepithelzellkarzinom der Lunge), Krebs des Peritoneums, hepatozellulärem Krebs, gastrischem Krebs oder Magenkrebs (einschließlich Magen-Darm-Krebs), Bauchspeicheldrüsenkrebs, Glioblastom, Gebärmutterhalskrebs, Eierstockkrebs, Leberkrebs, Blasenkrebs, Hepatom, Brustkrebs, Kolonkrebs, Kolorektalkrebs, Gebärmutterschleimhaut- oder Gebärmutterkrebs, Speicheldrüsenkarzinom, Nierenkrebs oder renalem Krebs, Leberkrebs, Prostatakrebs, Vulvakrebs, Schilddrüsenkrebs, Leberkarzinom, Kopf- und Halskrebs, B-Zell-Lymphom (einschließlich niedriggradigem/follikulärem Non-Hodgkin-Lymphom (NHL); kleinzelligem lymphozytischem (small lymphocytic, SL) NHL; mittelgradigem/follikulärem NHL; mittelgradigem diffusem NHL; hochgradigem immunoblastischem NHL; hochgradigem lymphoblastischem NHL; hochgradigem kleinzelligem NHL mit ungespaltenem Zellkern (small non-cleaved cell NHL); Bulky-Disease-NHL; Mantelzelllymphom; AIDS-bedingtem Lymphom und Waldenström-Makroglobulinämie); chronischer lymphatischer Leukämie (CLL); akuter lymphatischer Leukämie (ALL); Haarzellenleukämie; chronischer myeloblastischer Leukämie; Post-Transplantationslymphoproliferativer Störung (PTLD), anomaler Gefäßproliferation in Zusammenhang mit Phakomatosen, Ödem (wie dasjenige, das mit Gehirntumoren einhergeht) und Meigs-Syndrom; oder
(vii) Verwendung bei der Behandlung oder Vorbeugung einer Virusinfektion in einem Individuum, das dessen bedarf.

10. Zusammensetzung zur Verwendung nach Anspruch 9, wobei das Individuum Folgendes hat
(i) eine bakterielle Infektion, die durch mindestens ein Bakterium verursacht wird, ausgewählt aus der Gruppe, bestehend aus *Bordetella, Borrelia, Brucella, Burkholderia, Campylobacter, Chlamydia, Clostridium, Corynebacterium, Enterobacter, Enterococcus, Erwinia, Escherichia, Francisella, Haemophilus, Helicobacter, Legionella, Leptospira, Listeria, Mycobacterium, Mycoplasma, Neisseria, Pasteurella, Pelobacter, Pseudomonas, Rickettsia, Salmonella, Serratia, Shigella, Staphylococcus, Streptococcus, Treponema, Vibrio, Yersinia* und *Xanthomonas;*
(ii) eine Virusinfektion, die durch mindestens ein Virus verursacht wird, ausgewählt aus der Gruppe, bestehend aus *Adenoviridae, Papillomaviridae, Polyomaviridae, Herpesviridae, Poxviridae, Hepadnaviridae, Parvoviridae, Astroviridae, Caliciviridae, Picornaviridae, Coronaviridae, Flaviviridae, Retroviridae, Togaviridae, Arenaviridae, Bunyaviridae, Filoviridae, Orthomyxoviridae, Paramyxoviridae, Rhabdoviridae* und *Reoviridae;*
(iii) eine Virusinfektion, die durch ein Adenovirus, Herpes simplex Typ I-, Herpes simplex Typ 2-, Varicella-Zoster-Virus, Epstein-Barr-Virus, Cytomegalievirus, Herpesvirus Typ 8, Papillomvirus, BK-Virus, JC-Virus, Pocken-, Hepatitis B-, Bocavirus, Parvovirus B19, Astrovirus, Norwalk-Virus, Coxsackievirus, Hepatitis A-, Poliovirus, Rhinovirus, schweres akutes respiratorisches Syndrom-Virus, Hepatitis C-, Gelbfieber-, Dengue-Virus, West-Nil-Virus, Röteln-, Hepatitis E-, Humanes Immundefizienz-Virus (HIV), Influenza-, Guanarito-Virus, Junin-Virus, Lassa-Virus, Machupo-Virus, Sabia-Virus, Krim-Kongo-Hämorrhagisches Fieber-Virus, Ebola-Virus, Marburg-Virus, Masern-Virus, Mumps-Virus, Parainfluenza-, respiratorisches Syncytial-Virus, Humanes Metapneumovirus, Hendra-Virus, Nipah Virus, Tollwut-, Hepatitis D-, Rotavirus, Orbivirus, Coltivirus oder Banna-Virus verursacht wird;
(iv) eine Pilzinfektion, ausgewählt aus der Gruppe, bestehend aus Soor, Candidose, Kryptokokkose, Histoplasmose, Blastomykose, Aspergillose, Kokzidioidomykose, Parakokzidiomykose, Sporotrichose, Zygomykose, Chromoblastomykose, Lobomykose, Myzetom, Onychomykose, Piedra Pityriasis versicolor, Tinea barbae, Tinea capitis, Tinea corporis, Tinea cruris, Tinea favosa, Tinea nigra, Tinea pedis, Otomykose, Phaeohyphomykose oder Rhinosporidiose; oder
(v) eine Parasiteninfektion, die durch mindestens einen Parasiten verursacht wird, ausgewählt aus der Gruppe, bestehend aus *Entamoeba histolytica, Giardia lamblia, Cryptosporidium muris, Trypanosomatidae gambiense, Trypanosomatidae rhodesiense, Trypanosomatidae cruzi, Leishmania mexicana, Leishmania braziliensis, Leishmania tropica, Leishmania donovani, Toxoplasma gondii, Plasmodium vivax, Plasmodium ovale, Plasmodium malariae, Plasmodium falciparum, Trichomonas vaginalis, Histomonas meleagridis; Secementea; Trichuris trichiura, Ascaris lumbricoides, Enterobius vermicularis, Ancylostoma duodenale, Necator americanus, Strongyloides stercoralis, Wuchereria bancrofti, Dracunculus medinensis; Blutegeln, Leberegeln, Darmegeln, Lungenwürmern; Schistosoma mansoni,* Schistosoma *haematobium, Schistosoma japonicum, Fasciola hepatica, Fasciola gigantica, Heterophyes heterophyes* und *Paragonimus westermani.*

11. VISTA-Antagonist nach Anspruch 1 zur:
(i) Verwendung bei der Blockierung, Hemmung oder Neutralisierung von VISTA-vermittelter T-Zell-Suppression,
(ii) Verwendung bei der Stimulation einer Immunantwort in einem Individuum, das dessen bedarf, optional einem Individuum mit einer Infektion, ausgewählt aus der Gruppe, bestehend aus Bakterien-, Virus-, Parasiten- und Pilzinfektionen;
(iii) Verwendung bei der Behandlung von Krebs in einem Individuum, das dessen bedarf;
(iv) Verwendung bei der Verstärkung von Anti-Krebs- oder Anti-Tumor-Immunität in einem Individuum, das dessen bedarf;
(v) Verwendung bei der Behandlung oder Vorbeugung von Krebs, Hemmung der Tumorinvasion und/oder Krebsmetastasierung in einem Individuum, das dessen bedarf;
(vi) Verwendung nach (iii), (iv) oder (v), wobei der Krebs aus der Gruppe ausgewählt ist, bestehend aus Karzinom, Lymphom, Blastom, Sarkom, Leukämie, Lymphmalignomen, Melanom, Plattenepithelzellkrebs, Lungenkrebs (einschließlich kleinzelligem Lungenkrebs, nicht-kleinzelligem Lungenkrebs, Adenokarzinom der Lunge und Plattenepithelzellkarzinom der Lunge), Krebs des Peritoneums, hepatozellulärem Krebs, gastrischem Krebs oder Magenkrebs (einschließlich Magen-Darm-Krebs), Bauchspeicheldrüsenkrebs, Glioblastom, Gebärmutterhalskrebs, Eierstockkrebs, Leberkrebs, Blasenkrebs, Hepatom, Brustkrebs, Kolonkrebs, Kolorektalkrebs, Gebärmutterschleimhaut- oder Gebärmutterkrebs, Speicheldrüsenkarzinom, Nierenkrebs oder renalem Krebs, Leberkrebs, Prostatakrebs, Vulvakrebs, Schilddrüsenkrebs, Leberkarzinom, Kopf- und Halskrebs, B-Zell-Lymphom (einschließlich niedriggradigem/follikulärem Non-Hodgkin-Lymphom (NHL); kleinzelligem lymphozytischem (small lymphocytic, SL) NHL; mittelgradigem/follikulärem NHL; mittelgradigem diffusem NHL; hochgradigem immunoblastischem NHL; hochgradigem lymphoblastischem NHL; hochgradigem kleinzelligem NHL mit ungespaltenem Zellkern (small non-cleaved cell NHL); Bulky-Disease-NHL; Mantelzelllymphom; AIDS-bedingtem Lymphom und Waldenström-Makroglobulinämie); chronischer lymphatischer Leukämie (CLL); akuter lymphatischer Leukämie (ALL); Haarzellenleukämie; chronischer myeloblastischer Leukämie; Post-Transplantationslymphoproliferativer Störung (PTLD), anomaler Gefäßproliferation in Zusammenhang mit Phakomatosen, Ödem (wie dasjenige, das mit Gehirntumoren einhergeht) und Meigs-Syndrom; oder
(vii) Verwendung bei der Behandlung oder Vorbeugung einer Virusinfektion in einem Individuum, das dessen bedarf.

12. VISTA-Antagonist zur Verwendung nach Anspruch 11, ferner umfassend die Verwendung eines weiteren therapeutischen Mittels, wobei das VISTA-Antagonistenpeptid und das weitere Therapeutikum getrennt oder zusammen zur gleichen Zeit oder zu verschiedenen Zeitpunkten verabreicht werden können.

13. VISTA-Antagonist zur Verwendung nach Anspruch 12, wobei das weitere therapeutische Mittel ein Antikrebsmittel, ein antivirales oder anderes antiinfektiöses Mittel, ein Zytokin oder ein Immunagonist, wie CTLA-4-Ig, Anti-PD-1-, PD-L1- oder PD-L2-Fusionsproteine oder ein EGFR-Antagonist, ist.

14. Verwendung eines isolierten VISTA-Antagonisten bei einem Verfahren zur Kartierung des aktiven Zentrums von VISTA, umfassend: (a) Inkubieren eines isolierten VISTA-Fusionsproteins mit dem isolierten VISTA-Antagonisten, umfassend ein Peptid, das mit der Aminosäuresequenz von SEQ ID NO: 1 (Ser-Ser-Ala-Cys-Asp-Trp-Ile-Lys-Arg-Ser-Cys-His) identisch ist, oder umfassend ein Peptid mit einer Aminosäuresequenz, die sich von SEQ ID NO: 1 um höchstens 2 Aminosäurereste unterscheidet, oder ein Multimer, Konjugat, oder Derivat davon, wobei das Derivat davon eine Modifikation von einem oder mehreren Aminosäureresten oder eine Linkergruppe oder eine andere kovalent gebunden Gruppe enthält; und (b) Bestimmen der Bildungsstelle des isolierten VISTA-Antagonisten.

15. Verwendung nach Anspruch 14, wobei das aktive Zentrum von VISTA an einen VISTA-Rezeptor bindet und Immunsuppression vermittelt oder Schritt (b) Domänendeletion, Domänenaustausch, Aminosäuremutagenese, Footprinting, NMR, Röntgenkristallographie oder Homologiemodellierung umfasst.

## Revendications

1. Antagoniste de VISTA isolé choisi parmi les suivants :
(i) un antagoniste comprenant un peptide qui est identique à la séquence d'acides aminés de SEQ ID n° : 1 (Ser-Ser-Ala-Cys-Asp-Trp-Ile-Lys-Arg-Ser-Cys-His), ou un multimère, conjugué ou dérivé de celui-ci, le dérivé de celui-ci contenant une modification d'un ou plusieurs résidus d'acides aminés ou un groupe lieur ou autre groupe lié de manière covalente ;
(ii) un antagoniste comprenant un peptide qui est identique à la séquence d'acides aminés de SEQ ID n° : 1 (Ser-Ser-Ala-Cys-Asp-Trp-Ile-Lys-Arg-Ser-Cys-His) ou qui comprend un peptide ayant une séquence d'acides aminés qui diffère de SEQ ID n° : 1 d'au maximum 2 résidus d'acides aminés, ou un multimère, conjugué ou dérivé de celui-ci, le dérivé de celui-ci contenant une modification d'un ou plusieurs résidus d'acides aminés ou un groupe lieur ou autre groupe lié de manière covalente ;
(iii) un antagoniste qui comprend un peptide ayant une séquence d'acides aminés qui diffère de SEQ ID n° : 1 d'au maximum 1 résidu d'acide aminé, ou un multimère, conjugué ou dérivé de celui-ci, le dérivé de celui-ci contenant une modification d'un ou plusieurs résidus d'acides aminés ou un groupe lieur ou autre groupe lié de manière covalente ;
(iv) un antagoniste comprenant un peptide qui est identique à la séquence d'acides aminés de SEQ ID n° : 1 (Ser-Ser-Ala-Cys-Asp-Trp-Ile-Lys-Arg-Ser-Cys-His) ou un multimère ou conjugué de celui-ci ;
(v) un antagoniste qui est constitué d'un peptide qui est identique à la séquence d'acides aminés de SEQ ID n° : 1 (Ser-Ser-Ala-Cys-Asp-Trp-Ile-Lys-Arg-Ser-Cys-His) ;
(vi) un antagoniste selon l'un quelconque de (i) à (v) dans lequel les résidus de cystéine au niveau des positions 4 et 11 de SEQ ID n° : 1 (Ser-Ser-Ala-Cys-Asp-Trp-Ile-Lys-Arg-Ser-Cys-His), ou leur position correspondante dans un variant dudit peptide, forment un pont disulfure ;
(vii) un antagoniste selon l'un quelconque de (i) à (vi) qui a été modifié pour améliorer l'affinité de liaison et/ou la stabilité ;
(viii) un antagoniste selon (vii) dans lequel la modification est choisie dans le groupe constitué par le PEG, l'acétylation, le XTEN, l'albumine et la multimérisation ;
(ix) un antagoniste selon l'un quelconque de (i) à (viii) qui est directement ou indirectement attaché à un polypeptide d'immunoglobuline ou un fragment de celui-ci ;
(x) un antagoniste selon (ix) dans lequel ledit polypeptide d'immunoglobuline ou fragment de celui-ci comprend une région constante d'IgG1, d'IgG2, d'IgG3 ou d'IgG4 humaine ou un fragment de celle-ci ;
(xi) un antagoniste selon (x) dans lequel ledit polypeptide d'immunoglobuline comprend une région constante d'IgG1 humaine ou un fragment de celle-ci ;
(xii) un antagoniste selon l'un quelconque de (i) à (xi) qui comprend 2, 3, 4, 5, 6, 7 ou plus de 7 copies dudit peptide ;
(xiii) un antagoniste selon l'un quelconque de (i) à (xi) qui comprend une autre entité qui cible ledit peptide sur un site cible ;
(xiv) un antagoniste selon (xiii) dans lequel l'entité de ciblage est choisie entre un anticorps ou ligand qui se lie à un antigène, un récepteur exprimé par la cellule cible ou un agent infectieux ;
(xv) un antagoniste selon l'un quelconque de (i) à (xiv), l'antagoniste de VISTA étant attaché à une autre entité ou une autre copie dudit antagoniste de VISTA par l'intermédiaire d'un lieur ;
(xvi) un antagoniste selon (xv) dans lequel le lieur est un peptide qui permet à l'antagoniste de VISTA d'interagir avec VISTA exprimé sur la surface d'une cellule cible ;
(xvii) un antagoniste selon l'un quelconque de (i) à (xvi) qui est directement ou indirectement attaché à un marqueur détectable ou un agent thérapeutique ;
(xviii) un antagoniste selon l'un quelconque de (i) à (xvii) qui se lie au domaine extracellulaire de VISTA et perturbe son interaction avec un récepteur de VISTA ;
(xix) un antagoniste selon l'un quelconque de (i) à (xviii) qui réduit ou inhibe la suppression de lymphocytes T médiée par VISTA ; et/ou
(xx) un antagoniste selon l'un quelconque de (i) à (xix) qui induit une activité antitumorale et/ou antivirale.

2. Composition appropriée pour une utilisation thérapeutique, prophylactique ou diagnostique comprenant une quantité efficace du point de vue thérapeutique, prophylactique ou diagnostique d'un antagoniste de VISTA isolé selon la revendication 1.

3. Composition selon la revendication 2, comprenant en outre un vecteur, un diluant, un agent solubilisant ou un conservateur pharmaceutiquement acceptables ou un mélange de ceux-ci.

4. Composition selon la revendication 2 ou 3, comprenant en outre un autre agent thérapeutique.

5. Composition selon la revendication 4, dans laquelle l'autre agent thérapeutique est un agent anticancéreux, un agent antiviral, une cytokine ou un agoniste immun éventuellement choisi parmi les protéines de fusion d'Ig CTLA-4, d'anti-PD-1, de PD-L1 ou de PD-L2 et les antagonistes de l'EGFR.

6. Composition selon l'une quelconque des revendications 2 à 5, qui est appropriée pour une administration sous-cutanée ou une administration intraveineuse.

7. Séquence d'acide nucléique isolée codant pour un antagoniste de VISTA selon la revendication 1 ou vecteur ou cellule hôte contenant ladite séquence d'acide nucléique, ladite cellule hôte étant éventuellement une cellule de mammifère, une cellule bactérienne, une cellule fongique, une cellule de levure, une cellule aviaire ou une cellule d'insecte.

8. Procédé d'expression d'un antagoniste de VISTA comprenant la culture d'une cellule hôte selon la revendication 7 dans des conditions qui permettent l'expression dudit antagoniste de VISTA.

9. Composition comprenant une quantité efficace d'un antagoniste de VISTA selon la revendication 1 destinée à :
(i) être utilisée en blocage, inhibition ou neutralisation de la suppression de lymphocytes T médiée par VISTA ;
(ii) être utilisée en stimulation d'une réponse immunitaire chez un sujet qui en a besoin, éventuellement un sujet atteint d'une infection choisie dans le groupe constitué par les infections bactériennes, virales, parasitaires et fongiques ;
(iii) être utilisée en traitement d'un cancer chez un sujet qui en a besoin ;
(iv) être utilisée en renforcement d'immunité anticancéreuse ou antitumorale chez un sujet qui en a besoin ;
(v) être utilisée en traitement ou prévention d'un cancer, inhibition d'invasion tumorale et/ou de métastase cancéreuse chez un sujet qui en a besoin ;
(vi) être utilisée selon (iii), (iv) ou (v), le cancer étant choisi dans le groupe constitué par un carcinome, un lymphome, un blastome, un sarcome, une leucémie, les malignités lymphoïdes, un mélanome, un cancer à cellules squameuses, un cancer du poumon (notamment un cancer du poumon à petites cellules, un cancer du poumon non à petites cellules, un adénocarcinome du poumon et un carcinome du poumon à cellules squameuses), un cancer du péritoine, un cancer hépatocellulaire, un cancer gastrique ou de l'estomac (notamment un cancer gastro-intestinal), un cancer du pancréas, un glioblastome, un cancer du col de l'utérus, un cancer de l'ovaire, un cancer du foie, un cancer de la vessie, un hépatome, un cancer du sein, un cancer du côlon, un cancer colorectal, un carcinome de l'endomètre ou de l'utérus, un carcinome des glandes salivaires, un cancer du rein ou rénal, un cancer du foie, un cancer de la prostate, un cancer de la vulve, un cancer de la thyroïde, un carcinome hépatique, un cancer de la tête et du cou, un lymphome à cellules B (notamment un lymphome non hodgkinien (LNH) folliculaire/de bas grade ; un LNH à petits lymphocytes (LPL) ; un LNH folliculaire/de grade intermédiaire ; un LNH diffus de grade intermédiaire ; un LNH immunoblastique de haut grade ; un LNH lymphoblastique de haut grade ; un LNH à petites cellules non clivées de haut grade ; un LNH à forte masse tumorale ; un lymphome à cellules du manteau ; un lymphome lié au SIDA ; et la macroglobulinémie de Waldenström) ; une leucémie lymphocytaire chronique (LLC) ; une leucémie lymphoblastique aiguë (LLA) ; une leucémie à cellules chevelues ; une leucémie myéloblastique chronique ; un syndrome lymphoprolifératif post-greffe (SLPG), une prolifération vasculaire anormale associée à des phakomatoses, un œdème (tel que celui associé à des tumeurs du cerveau) et le syndrome de Meigs ; ou
(vii) être utilisée en traitement ou prévention d'une infection virale chez un sujet qui en a besoin.

10. Composition destinée à être utilisée selon la revendication 9, le sujet ayant
(i) une infection bactérienne provoquée par au moins une bactérie choisie dans le groupe constitué par *Bordetella, Borrelia, Brucella, Burkholderia, Campylobacter, Chlamydia, Clostridium, Corynebacterium, Enterobacter, Enterococcus, Erwinia, Escherichia, Francisella, Haemophilus, Helicobacter, Legionella, Leptospira, Listeria, Mycobacterium, Mycoplasma, Neisseria, Pasteurella, Pelobacter, Pseudomonas, Rickettsia, Salmonella, Serratia, Shigella, Staphylococcus, Streptococcus, Treponema, Vibrio, Yersinia* et *Xanthomonas ;*
(ii) une infection virale provoquée par au moins un virus choisi dans le groupe constitué par les *Adenoviridae, Papillomaviridae, Polyomaviridae, Herpesviridae, Poxviridae, Hepadnaviridae, Parvoviridae, Astroviridae, Caliciviridae, Picornaviridae, Coronoviridae, Flaviviridae, Retroviridae, Togaviridae, Arenaviridae, Bunyaviridae, Filoviridae, Orthomyxoviridae, Paramyxoviridae, Rhabdoviridae* et *Reoviridae ;*
(iii) une infection virale provoquée par un adénovirus, herpes simplex de type I, herpes simplex de type 2, le virus varicelle-zona, le virus d'Epstein-Barr, un cytomégalovirus, l'herpesvirus de type 8, un papillomavirus, le virus BK, le virus JC, le virus de la variole, le virus de l'hépatite B, le bocavirus, le parvovirus B19, un astrovirus, le virus de Norwalk, un virus Coxsackie, le virus de l'hépatite A, un poliovirus, un rhinovirus, le virus du syndrome respiratoire aigu sévère, le virus de l'hépatite C, le virus de la fièvre jaune, le virus de la dengue, le virus du Nil occidental, le virus de la rubéole, le virus de l'hépatite E, le virus de immunodéficience humaine (VIH), le virus de la grippe, le virus Guanarito, le virus Junin, le virus Lassa, le virus Machupo, le virus Sabia, le virus de la fièvre hémorragique de Crimée-Congo, le virus Ebola, le virus Marburg, le virus de la rougeole, le virus des oreillons, un virus parainfluenza, le virus syncytial respiratoire, un métapneumovirus humain, le virus Hendra, le virus Nipah, le virus de la rage, le virus de l'hépatite D, un rotavirus, un orbivirus, un coltivirus ou le virus Banna ;
(iv) une infection fongique choisie dans le groupe constitué par le muguet, la candidose, la cryptococcose, l'histoplasmose, la blastomycose, l'aspergillose, la coccidioïdomycose, la paracoccidioïdomycose, la sporotrichose, la zygomycose, la chromoblastomycose, la lobomycose, un mycétome, l'onychomycose, la piedra pityriasis versicolor, la teigne de la barbe, la teigne du cuir chevelu, la teigne du corps, la teigne de l'aine, la tinea favosa, la teigne noire, le pied d'athlète, l'otomycose, une phaeohyphomycose ou une rhinosporidiose ; ou
(v) une infection parasitaire provoquée par au moins un parasite choisi dans le groupe constitué par *Entamoeba histolytica, Giardia* lamblia, *Cryptosporidium muris, Trypanosomatidae gambiense, Trypanosomatidae rhodesiense, Trypanosomatidae cruzi, Leishmania mexicana, Leishmania braziliensis, Leishmania tropica, Leishmania donovani, Toxoplasma gondii, Plasmodium vivax, Plasmodium ovale, Plasmodium malariae, Plasmodium falciparum, Trichomonas vaginalis, Histomonas meleagridis ; Secementea ; Trichuris trichiura, Ascaris lumbricoides, Enterobius vermicularis, Ancylostoma duodenale, Necator americanus, Strongyloides stercoralis, Wuchereria bancrofti, Dracunculus medinensis ;* les bilharzioses, les douves du foie, les trématodes intestinaux, les douves du poumon ; *Schistosoma mansoni, Schistosoma haematobium, Schistosoma japonicum, Fasciola hepatica, Fasciola gigantica, Heterophyes heterophyes* et *Paragonimus westermani.*

11. Antagoniste de VISTA selon la revendication 1 destiné à :
(i) être utilisé en blocage, inhibition ou neutralisation de la suppression de lymphocytes T médiée par VISTA ;
(ii) être utilisé en stimulation d'une réponse immunitaire chez un sujet qui en a besoin, éventuellement un sujet atteint d'une infection choisie dans le groupe constitué par les infections bactériennes, virales, parasitaires et fongiques ;
(iii) être utilisé en traitement d'un cancer chez un sujet qui en a besoin ;
(iv) être utilisé en renforcement d'immunité anticancéreuse ou antitumorale chez un sujet qui en a besoin ;
(v) être utilisé en traitement ou prévention d'un cancer, inhibition d'invasion tumorale et/ou de métastase cancéreuse chez un sujet qui en a besoin ;
(vi) être utilisé selon (iii), (iv) ou (v), le cancer étant choisi dans le groupe constitué par un carcinome, un lymphome, un blastome, un sarcome, une leucémie, les malignités lymphoïdes, un mélanome, un cancer à cellules squameuses, un cancer du poumon (notamment un cancer du poumon à petites cellules, un cancer du poumon non à petites cellules, un adénocarcinome du poumon et un carcinome du poumon à cellules squameuses), un cancer du péritoine, un cancer hépatocellulaire, un cancer gastrique ou de l'estomac (notamment un cancer gastro-intestinal), un cancer du pancréas, un glioblastome, un cancer du col de l'utérus, un cancer de l'ovaire, un cancer du foie, un cancer de la vessie, un hépatome, un cancer du sein, un cancer du côlon, un cancer colorectal, un carcinome de l'endomètre ou de l'utérus, un carcinome des glandes salivaires, un cancer du rein ou rénal, un cancer du foie, un cancer de la prostate, un cancer de la vulve, un cancer de la thyroïde, un carcinome hépatique, un cancer de la tête et du cou, un lymphome à cellules B (notamment un lymphome non hodgkinien (LNH) folliculaire/de bas grade ; un LNH à petits lymphocytes (LPL) ; un LNH folliculaire/de grade intermédiaire ; un LNH diffus de grade intermédiaire ; un LNH immunoblastique de haut grade ; un LNH lymphoblastique de haut grade ; un LNH à petites cellules non clivées de haut grade ; un LNH à forte masse tumorale ; un lymphome à cellules du manteau ; un lymphome lié au SIDA ; et la macroglobulinémie de Waldenström), une leucémie lymphocytaire chronique (LLC) ; une leucémie lymphoblastique aiguë (LLA) ; une leucémie à cellules chevelues ; une leucémie myéloblastique chronique ; un syndrome lymphoprolifératif post-greffe (SLPG), une prolifération vasculaire anormale associée à des phakomatoses, un œdème (tel que celui associé à des tumeurs du cerveau) et le syndrome de Meigs ; ou
(vii) être utilisé en traitement ou prévention d'une infection virale chez un sujet qui en a besoin.

12. Antagoniste de VISTA destiné à être utilisé selon la revendication 11, comprenant en outre l'utilisation d'un autre agent thérapeutique, ledit peptide antagoniste de VISTA et l'autre agent thérapeutique pouvant être administrés séparément ou conjointement, en même temps ou à des moments différents.

13. Antagoniste de VISTA destiné à être utilisé selon la revendication 12, l'autre agent thérapeutique étant un agent anticancéreux, un agent antiviral ou autre agent anti-infectieux, une cytokine ou un agoniste immun tel que des protéines de fusion d'Ig CTLA-4, d'anti-PD-1, de PD-L1 ou de PD-L2 ou un antagoniste de l'EGFR.

14. Utilisation d'un antagoniste de VISTA isolé dans un procédé pour la cartographie du site actif de VISTA, comprenant :
(a) l'incubation d'une protéine de fusion de VISTA isolée avec l'antagoniste de VISTA isolé comprenant un peptide qui est identique à la séquence d'acides aminés de SEQ ID n° : 1 (Ser-Ser-Ala-Cys-Asp-Trp-Ile-Lys-Arg-Ser-Cys-His) ou qui comprend un peptide ayant une séquence d'acides aminés qui diffère de SEQ ID n° : 1 d'au maximum 2 résidus d'acides aminés ou un multimère, conjugué ou dérivé de celui-ci, le dérivé de celui-ci contenant une modification d'un ou plusieurs résidus d'acides aminés ou un groupe lieur ou autre groupe lié de manière covalente ; et
(b) la détermination du site de liaison de l'antagoniste de VISTA isolé.

15. Utilisation selon la revendication 14, dans laquelle le site actif de VISTA se lie à un récepteur de VISTA et médie la suppression immunitaire ou l'étape (b) comprend une délétion de domaine, une permutation de domaines, une mutagenèse d'acides aminés, une prise d'empreinte, une RMN, une cristallographie aux rayons X ou une modélisation par homologie.
